(19) Europäisches Patentamt
European Patent Office
Office européen des brevets

(11) EP 3 134 119 B1

(12) **EUROPEAN PATENT SPECIFICATION**

(45) Date of publication and mention of the grant of the patent:
**22.08.2018 Bulletin 2018/34**

(21) Application number: **15729222.8**

(22) Date of filing: **16.04.2015**

(51) Int Cl.:
***A61K 39/395*** $^{(2006.01)}$ ***G01N 33/574*** $^{(2006.01)}$

(86) International application number:
**PCT/IB2015/052796**

(87) International publication number:
**WO 2015/162532 (29.10.2015 Gazette 2015/43)**

(54) **CANCER TREATMENT**

KREBSBEHANDLUNG

TRAITEMENT ANTICANCÉREUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.04.2014 US 201461983951 P**

(43) Date of publication of application:
**01.03.2017 Bulletin 2017/09**

(73) Proprietor: **Pfizer Inc.**
**New York, NY 10017 (US)**

(72) Inventors:
- **MARTINI, Jean-Francois Andre**
  **Carlsbad, California 92009 (US)**
- **TARAZI, Jamal Christo**
  **San Diego, California 92124 (US)**
- **WILLIAMS, James Andrew**
  **San Diego, California 92121 (US)**

(74) Representative: **Pfizer**
**European Patent Department**
**23-25 avenue du Docteur Lannelongue**
**75668 Paris Cedex 14 (FR)**

(56) References cited:
- **B. I. RINI ET AL: "Diastolic Blood Pressure as a Biomarker of Axitinib Efficacy in Solid Tumors", CLINICAL CANCER RESEARCH, vol. 17, no. 11, 1 June 2011 (2011-06-01), pages 3841-3849, XP055199950, ISSN: 1078-0432, DOI: 10.1158/1078-0432.CCR-10-2806**
- **ROBERT J MOTZER ET AL: "Axitinib versus sorafenib as second-line treatment for advanced renal cell carcinoma: overall survival analysis and updated results from a randomised phase 3 trial", LANCET ONCOLOGY, vol. 14, 16 April 2013 (2013-04-16), pages 552-562, XP055199794,**
- **SUMANTA K PAL ET AL: "Tivozanib: current status and future directions in the treatment of solid tumors", EXPERT OPINION ON INVESTIGATIONAL DRUGS, vol. 21, no. 12, 1 December 2012 (2012-12-01), pages 1851-1859, XP055199814, ISSN: 1354-3784, DOI: 10.1517/13543784.2012.733695**
- **BRIAN I. RINI ET AL: "Axitinib in Metastatic Renal Cell Carcinoma: Results of a Pharmacokinetic and Pharmacodynamic Analysis", THE JOURNAL OF CLINICAL PHARMACOLOGY, vol. 53, no. 5, 1 May 2013 (2013-05-01), pages 491-504, XP055199818, ISSN: 0091-2700, DOI: 10.1002/jcph.73**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 134 119 B1

## Description

### FIELD

The field of the present disclosure involves molecular biology, oncology, and clinical diagnostics. The present invention as claimed provides a method of identifying a tumor that is sensitive to treatment with axitinib, and axitinib for use in the treatment of cancer using such a method.

### BACKGROUND

Most cancer drugs are effective in some patients, but not in others. This can be due to genetic variation among tumors, and can be observed even among tumors within the same patient. Variable patient response is particularly pronounced with respect to targeted therapeutics. Therefore, the full potential of targeted therapies cannot be realized without suitable tests for determining which patients will benefit from which drugs. According to the National Institutes of Health (NIH), the term "biomarker" is defined as "a characteristic that is objectively measured and evaluated as an indicator of normal biologic or pathogenic processes or pharmacological response to a therapeutic intervention."

The development of improved diagnostics based on the discovery of biomarkers has the potential to accelerate new drug development by identifying, in advance, those patients most likely to show a clinical response to a given drug. Such diagnostics have the potential to significantly reduce the size, length and cost of clinical trials. Technologies such as genomics, proteomics and molecular imaging currently enable rapid, sensitive and reliable detection of specific gene mutations, expression levels of particular genes, and other molecular biomarkers. Despite the availability of various technologies for molecular characterization of tumors, the clinical utilization of cancer biomarkers remains largely unrealized because relatively few cancer biomarkers have been discovered. For example, a recent review article states:

The challenge is discovering cancer biomarkers. Although there have been clinical successes in targeting molecularly defined subsets of several tumor types - such as chronic myeloid leukemia, gastrointestinal stromal tumor, lung cancer and glioblastoma multiforme - using molecularly targeted agents, the ability to apply such successes in a broader context is severely limited by the lack of an efficient strategy to evaluate targeted agents in patients. The problem mainly lies in the inability to select patients with molecularly defined cancers for clinical trials to evaluate these exciting new drugs. The solution requires biomarkers that reliably identify those patients who are most likely to benefit from a particular agent. (Sawyers, 2008, Nature 452:548-552, at 548.)

Comments such as the foregoing illustrate the recognition of a need for the discovery of clinically useful biomarkers and diagnostic methods based on such biomarkers.

There are three distinct types of cancer biomarkers: (1) prognostic biomarkers, (2) predictive biomarkers, and (3) pharmacodynamic (PD) biomarkers. A prognostic biomarker is used to classify a cancer, e.g., a solid tumor, according to aggressiveness, i.e., rate of growth and/or metastasis, and refractiveness to treatment. This is sometimes called distinguishing "good outcome" tumors from "poor outcome" tumors. A predictive biomarker is used to assess the probability that a particular patient will benefit from treatment with a particular drug. For example, patients with breast cancer in which the ERBB2 (HER2 or NEU) gene is amplified are likely to benefit from treatment with trastuzumab (HERCEPTIN®), whereas patients without ERBB2 gene amplification are unlikely to benefit from treatment with trastuzumab. A PD biomarker is an indication of the effect(s) of a drug on a patient while the patient is taking the drug. Accordingly, PD biomarkers often are used to guide dosage level and dosing frequency, during the early stages of clinical development of a new drug. For a discussion of cancer biomarkers, see, e.g., Sawyers, 2008, Nature 452:548-552. Rini et al, Clinical Cancer Research, 17(11), 3841-9 (1 June 2011) discloses diastolic blood pressure as a biomarker of axitinib efficacy in solid tumors.

Axitinib (also known as Inlyta®) is an orally administered small-molecule receptor tyrosine kinase inhibitor that acts on vascular endothelial growth factor receptors (VEGFRs). Axitinib is thought to reduce tumor growth and metastasis by inhibiting angiogenesis, and to reduce tumor growth and cause regression by acting directly on cells that express, and are dependent on, these receptors. Axitinib is approved multi-nationally for the treatment of metastatic renal cell cancer (mRCC) after disease progression on, or resistance to, cytokines or sunitinib (also known as Sutent®).

Despite a large amount of pre-clinical and clinical research focused on VEGFR inhibitors, the mechanisms responsible for the anti-tumor activity of such inhibitors are not completely understood. In particular, the role of tumor infiltrating lymphocytes in influencing mRCC patients’ prognosis and sensitivity/resistance to anti-angiogenic agents is not fully understood (e.g. see Polimeno et al., 2013, BJU Int 112:686-696). As with other types of targeted therapy, some, but not all, patients benefit from axitinib therapy. Therefore, there is a need for diagnostic methods based on predictive biomarkers that can be used to identify patients with tumors that are likely (or unlikely) to respond to treatment with axitinib.

## SUMMARY

As will be discussed in more detail herein, the present disclosure relates in part to the finding that tumor myeloid (i.e. cluster of differentiation 68 "CD68") infiltration (e.g., elevated CD68 levels in terms of the percentage of CD68-positive cells and the density of CD68-positive cells) in a tissue sample from a mammalian tumor correlates with improved progression free survival with axitinib. Accordingly, the present disclosure provides methods of identifying a tumor that is more likely to respond positively to treatment with axitinib, and to axitinib for use in the treatment of subjects with tumors that have been identified as being more likely to respond to axitinib.

For example, in one embodiment, the disclosure relates to a method of identifying a tumor that is sensitive to treatment with axitinib, comprising: (a) measuring CD68 polypeptide expression level in a tissue sample from a tumor obtained from a human patient being considered for treatment with a axitinib; and (b) comparing the CD68 expression level in step (a) against a threshold CD68 expression level determined by measuring CD68 polypeptide expression in tissue samples of tumors obtained from human patients previously treated with the axitinib and shown to be resistant to the axitinib and human patients previously treated with axitinib and shown to be sensitive to the axitinib, wherein a CD68 expression level above the threshold level indicates that the tumor is sensitive to treatment with the axitinib. In a further embodiment, the step of measuring CD68 polypeptide expression is performed by immunohistochemistry. In a further embodiment, the step of measuring CD68 polypeptide expression by immunohistochemistry is carried out by image analysis from a whole slide scan, where the percentage of CD68-positive cells in the sample is determined. In a further embodiment, such methods further comprise the step of determining the density of CD68-positive cells in the sample. In a further embodiment the tumor in any of such methods is selected from the group consisting of a breast tumor, a lung tumor, a kidney tumor, a colorectal tumor, and a pancreatic tumor.

In a further embodiment, the present disclosure provides axitinib for use in the treatment of mRCC comprising administration of axitinib to a patient determined to have a mRCC tumor that is sensitive to axitinib according any of the methods described herein.

In a further embodiment the present disclosure provides axitinib for use in the treatment of cancer, comprising: a) determining the percentage of CD68-positive cells in a tumor from a subject; and b) administration of axitinib to the subject if said percentage is at least 2%, at least 3%, at least 4%, at least 4.5%, at least 4.6%, at least 4.7%, at least 4.8%, at least 4.9%, at least 5.0%, at least 5.1%, at least 5.2%, at least 5.3%, at least 5.4%, at least 5.5%, at least 5.6%, at least 5.7%, at least 5.8%, at least 5.9%, at least 6.0%, at least 6.5%, at least 7.0%, at least 8%, at least 9%, at least 10%, at least 15%, or at least 20%.

In a further embodiment, the present disclosure provides axitinib for use in the treatment of cancer, comprising: a) determining the cell density of CD68-positive cells in a tumor from a subject; and b) administration of axitinib to the subject if said cell density is at least 0.05 cells/$mm^2$, at least 0.06 cells/$mm^2$, at least 0.07 cells/$mm^2$, at least 0.08 cells/$mm^2$, at least 0.09 cells/$mm^2$, at least 1.0 cells/$mm^2$, at least 1.1 cells/$mm^2$, at least 1.2 cells/$mm^2$, at least 1.3 cells/$mm^2$, at least 1.4 cells/$mm^2$, or at least 1.5 cells/$mm^2$.

In a further embodiment, the present disclosure provides axitinib for use in the treatment of cancer comprising administration of axitinib to a subject with a tumor, wherein at least 2%, at least 3%, at least 4%, at least 4.5%, at least 4.6%, at least 4.7%, at least 4.8%, at least 4.9%, at least 5.0%, at least 5.1%, at least 5.2%, at least 5.3%, at least 5.4%, at least 5.5%, at least 5.6%, at least 5.7%, at least 5.8%, at least 5.9%, at least 6.0%, at least 6.5%, at least 7.0%, at least 8%, at least 9%, at least 10%, at least 15%, or at least 20% of the cells in said tumor are CD68-positive.

In a further embodiment, the present disclosure provides axitinib for use in the treatment of cancer comprising administration of axitinib to a subject with a tumor, wherein the cell density of CD68-positive cells in said tumor is at least 0.05 cells/$mm^2$, at least 0.06 cells/$mm^2$, at least 0.07 cells/$mm^2$, at least 0.08 cells/$mm^2$, at least 0.09 cells/$mm^2$, at least 1.0 cells/$mm^2$, at least 1.1 cells/$mm^2$, at least 1.2 cells/$mm^2$, at least 1.3 cells/$mm^2$, at least 1.4 cells/$mm^2$, or at least 1.5 cells/$mm^2$.

In a further embodiment, the present disclosure provides axitinib for use in the treatment of cancer comprising: a) determining the percentage of CD68-positive cells in a tumor from a subject; b) determining the cell density of CD68-positive cells in the tumor; and c) administration of axitinib to the subject if said percentage is at least 2%, at least 3%, at least 4%, at least 4.5%, at least 4.6%, at least 4.7%, at least 4.8%, at least 4.9%, at least 5.0%, at least 5.1%, at least 5.2%, at least 5.3%, at least 5.4%, at least 5.5%, at least 5.6%, at least 5.7%, at least 5.8%, at least 5.9%, at least 6.0%, at least 6.5%, at least 7.0%, at least 8%, at least 9%, at least 10%, at least 15%, or at least 20%; and said cell density is at least 0.05 cells/$mm^2$, at least 0.06 cells/$mm^2$, at least 0.07 cells/$mm^2$, at least 0.08 cells/$mm^2$, at least 0.09 cells/$mm^2$, at least 1.0 cells/$mm^2$, at least 1.1 cells/$mm^2$, at least 1.2 cells/$mm^2$, at least 1.3 cells/$mm^2$, at least 1.4 cells/$mm^2$, or at least 1.5 cells/$mm^2$.

In a further embodiment, the present disclosure provides axitinib for use in the treatment of cancer comprising administration of axitinib to a subject with a tumor, wherein at least 2%, at least 3%, at least 4%, at least 4.5%, at least 4.6%, at least 4.7%, at least 4.8%, at least 4.9%, at least 5.0%, at least 5.1%, at least 5.2%, at least 5.3%, at least 5.4%, at least 5.5%, at least 5.6%, at least 5.7%, at least 5.8%, at least 5.9%, at least 6.0%, at least 6.5%, at least 7.0%, at

least 8%, at least 9%, at least 10%, at least 15%, or at least 20% of the cells in said tumor are CD68-postive; and wherein the cell density of CD68-positive cells in said tumor is at least 0.05 cells/$mm^2$, at least 0.06 cells/$mm^2$, at least 0.07 cells/$mm^2$, at least 0.08 cells/$mm^2$, at least 0.09 cells/$mm^2$, at least 1.0 cells/$mm^2$, at least 1.1 cells/$mm^2$, at least 1.2 cells/$mm^2$, at least 1.3 cells/$mm^2$, at least 1.4 cells/$mm^2$, or at least 1.5 cells/$mm^2$.

In a further embodiment, the present disclosure provides any of the methods and uses disclosed herein, wherein said tumor is selected from the group consisting of a breast tumor, a lung tumor, a kidney tumor, a colorectal tumor, and a pancreatic tumor. In one embodiment, the present disclosure provides any of the methods and uses disclosed herein, wherein the cancer or tumor is mRCC.

In further embodiments, the methods and uses disclosed herein are carried out wherein the step of measuring the percentage or cell density of CD68 positive cells is performed using immunohistochemistry, and further wherein the use of image analysis from a whole slide scan from a tumor sample is employed.

In some embodiments of the present disclosure, measuring macrophage content is performed by measuring the presence or an amount of a macrophage marker protein. In other embodiments, measuring macrophage content is performed by determining the number of macrophages in a given cell population. For example, measuring macrophage content can be performed by immunohistochemistry involving detection of a macrophage marker protein. In another embodiment, measuring macrophage content is performed by measuring the presence or an amount of mRNA encoding a macrophage marker protein. Examples of macrophage marker proteins include CCR2, CD14, CD68, CD163, CSF1R and MSR1. The threshold determination analysis can include a receiver operator characteristic curve analysis. Methods of the present disclosure are useful for testing various types of tumors, including, e.g., breast tumors, lung tumors, kidney tumors, colorectal tumors, and pancreatic tumors.

## BRIEF DESCRIPTION OF THE DRAWINGS

Figure 1 shows the demographic and baseline characteristics of the subjects that were included in the A4061032 study.

Figure 2 shows a summary of the percent and density of positive cells for biomarkers CD3 and CD68 by slides versus blocks that were collected as part of the A4061032 study.

Figure 3 shows a Kaplan-Meier plot of PFS for comparison of less than median and greater than or equal to median values of percent and density of positive cells for the biomarker CD68.

Figure 4 shows a Kaplan-Meier plot of PFS for comparison of less than median and greater than or equal to median values of percent and density of positive cells for the biomarker CD68 in patients with prior Sutent® treatment.

Figure 5 shows a Kaplan-Meier plot of PFS for comparison of less than median and greater than or equal to median values of percent and density of positive cells for the biomarker CD3.

Figure 6 shows a Kaplan-Meier plot of PFS for comparison of less than median and greater than or equal to median values of percent and density of positive cells for the biomarker CD3 in patients with prior Sutent® treatment.

Figure 7 shows a summary of OS by less than and greater than or equal to median cut point stratum for each biomarker CD3 and CD68 percent and density of positive cells.

Figure 8 shows a summary of OS by less than and greater than or equal to median cut point stratum for each biomarker CD3 and CD68 percent and density of positive cells in patients with prior Sutent® treatment.

Figure 9 shows a Kaplan-Meier plot of OS for comparison of less than greater than or equal to median for biomarkers CD3 and CD68 for percent and density of positive cells in patients with prior Sutent® treatment.

Figure 10 shows a summary of the CD3 and CD68 biomarkers, percent and density of positive cells by response category.

Figure 11 shows a summary of the CD3 and CD68 biomarkers, percent and density of positive cells by response category in patients with prior Sutent® treatment.

## DETAILED DESCRIPTION

Definitions

As used herein, Inlyta®, "AG-13736" and "axitinib" mean 6-[2-(methylcarbamoyl)phenylsulfanyl]-3-E-[2-(pyridin-2-yl)ethenyl]indazole, which has the following chemical structure, including salts and polymorphs thereof:

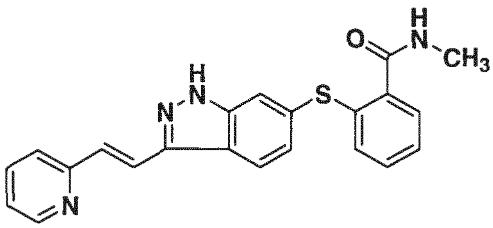

As used herein, "macrophage marker protein" means a macrophage cell surface protein, the detection of which is useful for identifying macrophages among the other types of cells present in a tissue sample from a tumor. Exemplary human macrophage marker proteins are CCR2, CD14, CD68, CD163, CSFIR and MSRI. Other macrophage marker proteins can be employed in practicing the present disclosure.

As used herein, "receiver operating characteristic" (ROC) curve means a plot of false positive rate (sensitivity) versus true positive rate (specificity) for a binary classifier system. In construction of an ROC curve, the following definitions apply:

False negative rate "FNR" = 1 - TPR

True positive rate "TPR" = true positive / (true positive + false negative)

False positive rate "FPR" = false positive / (false positive + true negative)

As used herein, "response" or "responding" to treatment means, with regard to a treated tumor, that the tumor displays: (a) slowing of growth, (b) cessation of growth, or (c) regression.

As used herein, "threshold determination analysis" means analysis of a dataset representing a given tumor type, e.g., human renal cell carcinoma, to determine a threshold score for that particular tumor type. The dataset representing a given tumor type can include, for each tumor from a group of such tumors: (a) actual tumor response data (response and non-response to a treatment such as axitinib), and (b) macrophage content and/or CD68 expression levels.

As used herein, "threshold score" means a score above which a tumor is classified as being likely sensitive to treatment, such as with axitinib.

As used herein, "CD68 expression level" or "CD68 polypeptide expression level" means the level of CD68 protein that is expressed in a tumor sample, and can be determined by any appropriate analytical technique such as immunohistochemistry. Furthermore, the "CD68 expression level" can be expressed in a variety of terms, including the percentage of cells in a given sample that are determined to be "CD68-positive", and the density of cells in a given sample that are determined to be "CD68-positive".

As used herein, "CCR2" (chemokine (C-C motif) receptor 2 also known as CD 192, CKR2, CMKBR2, MCP-I-R, CC-CKR-2, FLJ78302, MGC103828, MGC111760, and MGC168006) means the human protein encoded by the gene identified by Entrez GeneID No. 729230 and allelic variants thereof.

As used herein, "CD14" means the human protein encoded by the gene identified by Entrez GeneID No. 929 and allelic variants thereof.

As used herein, "CD68" (also known as GP110; SCARD1; and DKFZp686M 18236) means the human protein encoded by the gene identified by Entrez GeneID No. 968 and allelic variants thereof.

As used herein, a "CD68-positive" cell is a cell wherein the presence of CD68 is detected by any appropriate analytical technique, such as immunohistochemistry.

As used herein, "CD163" (also known as MI 30 and MM 130) means the human protein encoded by the gene identified by Entrez GeneID No. 9332 and allelic variants thereof.

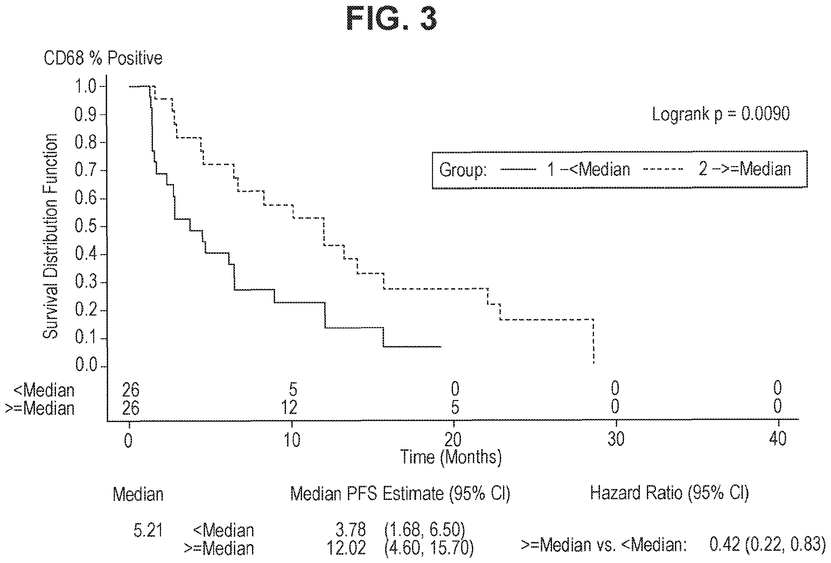

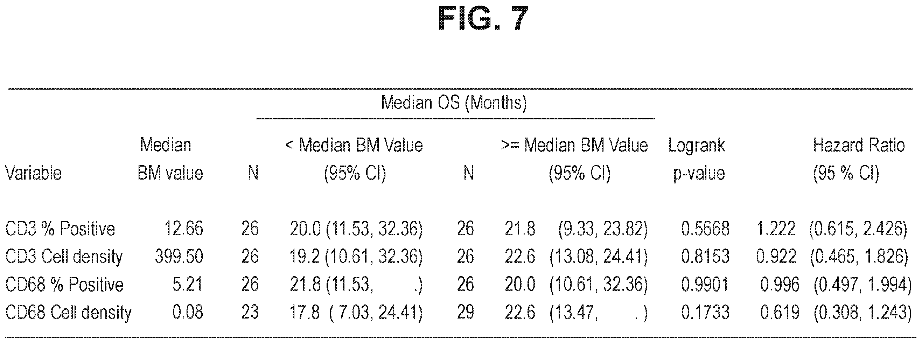

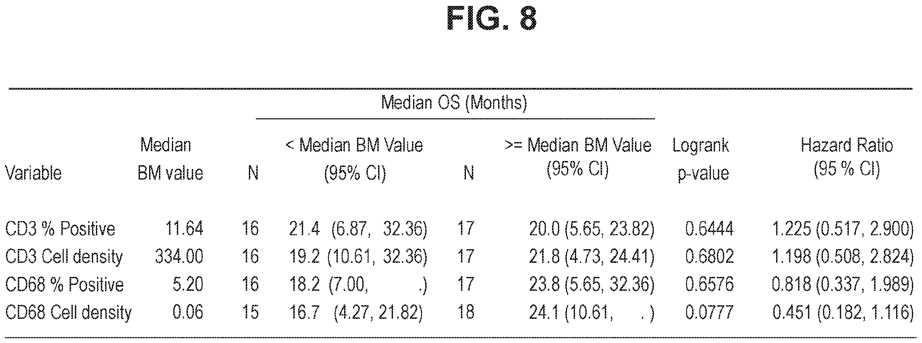

As used herein, "CSF1R" (colony stimulating factor 1 receptor also known as CSFR, FMS, FIM2, C-FMS, and CD115) means the human protein encoded by the gene identified by Entrez GeneID No. 1436 and allelic variants thereof.
As used herein, "MSR1" (macrophage scavenger receptor 1 also called CD204, SCARA1, SR-A, phSRI and phSR2) means the human protein encoded by the gene identified by Entrez GeneID No. 4481 and allelic variants thereof.
As used herein, "HR" means hazard ratio. In Figures 3, 4, 5, 6 and 9, assuming proportional hazards, a hazard ratio greater than 1 indicates a reduction in hazard rate in favor of < Median; a hazard ratio less than 1 indicates a reduction in hazard rate in favor of >= Median; and logrank p-values were produced only when N>=10 in both comparison groups. In Figures 7 and 8, assuming proportional hazards, a hazard ratio greater than 1 indicates a reduction in hazard rate in favor of < Median; a hazard ratio less than 1 indicates a reduction in hazard rate in favor of >= Median; and logrank p-value was produced only when N>=10 in both comparison groups; and hazard ratio statistic was produced only when N>=5 in both comparison groups; however, logrank p-value and hazard ratio were removed when zero event was observed in either group.

Clinical Studies

Inlyta®, hereafter referred to as axitinib, is an orally administered small-molecule receptor tyrosine kinase inhibitor that acts on vascular endothelial growth factor receptors (VEGFRs). Axitinib is expected to reduce tumor growth and metastasis by inhibiting angiogenesis, and to reduce tumor growth and cause regression by acting directly on cells that express, and are dependent on these receptors. Axitinib is approved multi-nationally for the treatment of metastatic renal cell cancer (mRCC) after disease progression on, or resistance to, cytokines or sunitinib.
Study A4061032 (ClinicalTrials.gov Identifier: NCT00678392) was a phase 3 registrational trial entitled "Axitinib (AG-013736) as second line therapy for metastatic renal cell cancer: Axis trial". The trial was designed to demonstrate that axitinib is superior to sorafenib in delaying tumor progression in patients with mRCC after failure of one first line regimen. A total of 650 patients were planned to be enrolled, and 723 subjects were eventually enrolled in the study.
As described in further detail in the Examples, formalin-fixed paraffin embedded (FFPE) tumor samples were collected from patients who participated in A4061032, and who provided specific consent for collection of tumor sample. Tumor myeloid (cluster of differentiation 68 "CD68") or lymphocyte (cluster of differentiation 3 "CD3") infiltration was assessed by immunohistochemistry (IHC) in tumor samples from 52 axitinib-treated patients. The aim was to investigate the potential association of these biomarkers with efficacy, consistent with the hypothesis that myeloid infiltration confers resistance to anti-angiogenic agents targeting the VEGF-VEGFR2 pathway (see Shojaei et al. (2007) Nat. Biotechnol. 25(8):911-920; and Lin et al. (2010) Eur. J. Cancer Suppl. 8(7):191).
The evaluation of CD3 and CD68 was performed by image analysis from a whole slide scan. The region of interest was circled, and an image analysis algorithm was run. The percentage of positive cells (number of positive cells/total number of cells) and the density of positive cells (e.g. number of positive cells/$mm^2$) was measured. Some patients donated FFPE blocks, and some donated slides cut from blocks. Samples from all patients were analyzed, whether they were provided as slides or blocks.
There were 52 evaluable patients for IHC data, 33 of which were previously treated with Sutent® (suntinib). There were no correlations between CD3 data and any of the endpoints measured. As described in further detail in the Examples, for CD68 the percentage of positive cells and cell density were closely correlated and were two-fold higher in patients with an objective response versus non-responders.
Regardless of prior treatment, median progression free survival (PFS) in patients with ≥ median CD68 values (cut off = 5.21 % positive cells or cell density of 0.08 cells/$mm^2$) was 12.0 months for both cutpoints vs 3.7 and 3.8 months respectively for patients with <median biomarker values (hazard ratio [HR]=0.42, logrank p-value≤50.01). For patients pre-treated with Sutent® a similar trend with PFS was observed with marginal statistical significance (p-values: 0.066 and 0.056 for % positive cells and cell density, respectively). Similar trends of favorable efficacy were observed for objective response and overall survival (OS) for patients with relatively higher CD68 cell count, although these differences were not statistically significant when all patients were assessed together or when only Sutent® pre-treated patients were assessed.
Additional receiver operating characteristics (ROC) analyses were conducted to better understand the sensitivity and specificity of baseline tumor CD68 levels, and to optimize definition of CD68 cut-points from the initial selected median CD68 values. Default cut-off values of 2, 4, 6 and 8 months PFS were selected, and again patients with higher percentage of CD68 positive cells and cell density were observed to have longer values for PFS (or equivalently less chance of disease progression or death) at each of the four PFS time points.
As described in greater detail in the Examples, the highest observed sensitivity, specificity and area under the curve (AUC) values were observed at 2 months PFS, where the AUCs of the ROC curve were 0.776 and 0.809 for percentage of CD68 positive cells and cell density, respectively, which indicate compelling overall diagnostic accuracy for PFS using CD68 expression levels. The optimal cut-off points for predicting PFS at 6 months were 4.41% and 0.06 cells/$mm^2$ for CD68 percent and density of positive cells, respectively. Values were similar for patients pre-treated with

Sutent®.

ROC analysis was also used for assessment of CD68 versus ORR. As described in greater detail in the Examples, the AUCs were 0.791 and 0.784 for percentage of CD68 positive cells and cell density, respectively, which indicate again compelling overall accuracy for predicting ORR using the CD68 expression levels. The optimal cut off points for predicting ORR were 9.42% and 0.13 cells/$mm^2$ for percentage of CD68 positive cells and cell density, respectively. Values were similar for patients pre-treated with Sutent®. For survival probability at 21 months, the median value for the trial, ROC analysis did not show a statistically significant association, with an AUC of 0.559.

In conclusion, regardless of previous treatment, favorable PFS was observed for patients with higher tumor CD68 levels. Median PFS in patients with >median CD68 values was 12.0 months for both cutpoints versus 3.7 and 3.8 months for patients with <median biomarker values (HR=0.42, logrank p-value≤0.01). ROC analysis indicated compelling predictive value for this data at 2, 4, 6 and 8 months, and refined cut off points (at 6 months - 4.41% and 0.06 cells/$mm^2$ for percentage of CD68 positive cells and cell density).

Accordingly, the present disclosure relates to the finding that higher CD68 expression is associated with higher ORR and longer PFS for patients treated with a VEGFR inhibitor, such as axitinib. The absence of association with OS may be due to confounding post-progression treatments after progression on axitinib. These observations are consistent with a mechanism of increased VEGF production and associated angiogenic status with higher macrophage infiltration, and therefore greater sensitivity to the treatment effects of axitinib. ROC analysis of PFS, the registrational endpoint, showed greatest sensitivity and specificity after two months of treatment.

CD68 expression has previously been reported to associate with outcome for mRCC patients receiving tivozanib treatment (Lin et al. (2010) Eur. J. Cancer Suppl. 8(7):191). Myeloid (CD11b Gr+) cells have also previously been shown to confer resistance to bevacizumab in a lung animal model (Shojaei et al. (2007) Nat Biotechnol. 25(8):911-920). This data would be consistent with poorer outcomes for patients with higher CD68 tumor levels. However this was not observed in this study.

For RCC patients, significant progress has been made in the identification of *prognostic* biomarkers, but no *predictive* markers of efficacy have been identified for targeted VEGFR inhibitors such as axitinib. According to a recent review (Tonini G et al. (2011) Exper Rev Anticancer Ther 11(6):921-930), the choice of the most appropriate therapy for RCC patients is still dependent on risk criteria (MSKCC) and other prognostic criteria. Furthermore, the authors state that these combined criteria provide information on RCC patient outcome, and that predictive factors of response to therapy for mRCC are needed. There is a need for validation of potential markers in randomized clinical trials (*Id*.). Standardization of tissue collection and analysis is also cited as a major challenge in developing molecular biomarkers to potentially guide therapy (Sonpavde G and Choueiri T, (2012) Br J Cancer 107(7):1009-1016).

Methods and analytical techniques that can be used in carrying out the present disclosure are further disclosed below.

## Tissue Sample

A tissue sample from a tumor in a human patient can be used as a source of RNA, a source of protein, or a source of thin sections for immunohistochemistry (IHC), so level of CD68 expression in the sample can be determined as described in the present disclosure. The tissue sample can be obtained by using conventional tumor biopsy instruments and procedures. Endoscopic biopsy, excisional biopsy, incisional biopsy, fine needle biopsy, punch biopsy, shave biopsy and skin biopsy are examples of recognized medical procedures that can be used by one of skill in the art to obtain tumor samples. The tumor tissue sample should be large enough to provide sufficient RNA, protein, or thin sections for measuring marker gene, e.g., CD68 expression level or visualizing macrophages by IHC, e.g., CD68-positive cell expression.

The tumor tissue sample can be in any form that allows measurement of macrophage content, or specifically CD68. In other words, the tissue sample must be sufficient for RNA extraction, protein extraction, or preparation of thin sections. Accordingly, the tissue sample can be fresh, preserved through suitable cryogenic techniques, or preserved through non-cryogenic techniques. A standard process for handling clinical biopsy specimens is to fix the tissue sample in formalin and then embed it in paraffin. Samples in this form are commonly known as formalin-fixed, paraffin-embedded (FFPE) tissue. Suitable techniques of tissue preparation for subsequent analysis are well-known to those of skill in the art.

## Macrophage Content

In practicing the present disclosure, determining the level of macrophage content (e.g., macrophage number or expression of a macrophage marker such as CD68, e.g., expression of a macrophage marker protein or expression of a mRNA encoding a macrophage marker protein such as CD68) in a tissue sample (e.g., from a tumor) can be performed by any suitable method, of which there are several. For example, measuring macrophage content indirectly can be done by measuring the expression of one or more genes known to be useful as macrophage markers, such as

CD68. Various methods for measuring gene expression are known in the art. Such methods can be applied in determining the level of macrophage marker proteins or mRNA encoding macrophage marker proteins. Exemplary human macrophage marker genes are CCR2, CD14, CD68, CD163, CSF1R and MSR1. Other macrophage markers can be used, as well.

RNA Analysis

Conventional microarray analysis and quantitative polymerase chain reaction (QPCR) are examples of methods for determining the level of macrophage marker gene expression at the mRNA level. In some embodiments of the disclosure, RNA is extracted from the cells, tumor or tissue of interest using standard protocols. In other embodiments, RNA analysis is performed using techniques that do not require RNA isolation.

RNA Isolation

Methods for rapid and efficient extraction of eukaryotic mRNA, i.e., poly(a) RNA, from tissue samples are well established and known to those of skill in the art. See, e.g., Ausubel et al., 1997, Current Protocols of Molecular Biology, John Wiley and Sons. The tissue sample can be fresh, frozen or fixed paraffin-embedded (FFPE) samples such as clinical study tumor specimens. In general, RNA isolated from fresh or frozen tissue samples tends to be less fragmented than RNA from FFPE samples. FFPE samples of tumor material, however, are more readily available, and FFPE samples are suitable sources of RNA for use in methods of the present disclosure. For a discussion of FFPE samples as sources of RNA for gene expression profiling by RT-PCR, see, e.g., Clark-Langone et al., 2007, BMC Genomics 8:279. Also see, De Andres et al., 1995, Biotechniques 18:42044; and Baker et al., U.S. Patent Application Publication No. 2005/0095634. The use of commercially available kits with vendor's instructions for RNA extraction and preparation is widespread and common. Commercial vendors of various RNA isolation products and complete kits include Qiagen (Valencia, CA), Invitrogen (Carlsbad, CA), Ambion (Austin, TX) and Exiqon (Woburn, MA).

In general, RNA isolation begins with tissue/cell disruption. During tissue/cell disruption it is desirable to minimize RNA degradation by RNases. One approach to limiting RNase activity during the RNA isolation process is to ensure that a denaturant is in contact with cellular contents as soon as the cells are disrupted. Another common practice is to include one or more proteases in the RNA isolation process. Optionally, fresh tissue samples are immersed in an RNA stabilization solution, at room temperature, as soon as they are collected. The stabilization solution rapidly permeates the cells, stabilizing the RNA for storage at 4 degrees centigrade, for subsequent isolation.

In some protocols, total RNA is isolated from disrupted tumor material by cesium chloride density gradient centrifugation. In general, mRNA makes up approximately 1 percent to 5 percent of total cellular RNA. Immobilized Oligo(dT), e.g., oligo(dT) cellulose, is commonly used to separate mRNA from ribosomal RNA and transfer RNA. If stored after isolation, RNA must be stored in under RNase-free conditions. Methods for stable storage of isolated RNA are known in the art. Various commercial products for stable storage of RNA are available.

Microarray

The mRNA expression level of one or more genes encoding macrophage marker proteins such as CD68 can be measured using conventional DNA microarray expression profiling technology. A DNA microarray is a collection of specific DNA segments or probes affixed to a solid surface or substrate such as glass, plastic or silicon, with each specific DNA segment occupying a known location in the array. Hybridization with a sample of labeled RNA, usually under stringent hybridization conditions, allows detection and quantitation of RNA molecules corresponding to each probe in the array. After stringent washing to remove non-specifically bound sample material, the microarray is scanned by confocal laser microscopy or other suitable detection method. Modern commercial DNA microarrays, often known as DNA chips, typically contain tens of thousands of probes, and thus can measure expression of tens of thousands of genes simultaneously. Such microarrays can be used in practicing the present disclosure. Alternatively, custom chips containing as few probes as those needed to measure expression of one or more genes encoding macrophage marker proteins, such as CD68, plus necessary controls or standards, e.g., for data normalization, can be used in practicing the disclosure.

To facilitate data normalization, a two-color microarray reader can be used. In a two-color (two-channel) system, samples are labeled with a first fluorophore that emits at a first wavelength, while an RNA or cDNA standard is labeled with a second fluorophore that emits at a different wavelength. For example, Cy3 (570 nm) and Cy5 (670 nm) often are employed together in two-color microarray systems.

DNA microarray technology is well-developed, commercially available, and widely employed. Therefore, in performing methods disclosed herein, a person of ordinary skill in the art can use microarray technology to measure expression levels of genes encoding macrophage marker proteins such as CD68 without undue experimentation. DNA

microarray chips, reagents (such as those for RNA or cDNA preparation, RNA or cDNA labeling, hybridization and washing solutions), instruments (such as microarray readers) and protocols are well known in the art and available from various commercial sources. Commercial vendors of microarray systems include Agilent Technologies (Santa Clara, CA) and Affymetrix (Santa Clara, CA), but other PCR systems can be used as well.

Quantitative RT-PCR

The level of mRNA representing individual genes encoding macrophage marker proteins such as CD68 can be measured using conventional quantitative reverse transcriptase polymerase chain reaction (qRT-PCR) technology. Advantages of qRT-PCR include sensitivity, flexibility, quantitative accuracy, and ability to discriminate between closely related mRNAs. Guidance concerning the processing of tissue samples for quantitative PCR is available from various sources, including manufacturers and vendors of commercial products for qRT-PCR (e.g., Qiagen (Valencia, CA) and Ambion (Austin, TX)). Instrument systems for automated performance of qRT-PCR are commercially available and used routinely in many laboratories. An example of a well-known commercial system is the Applied Biosystems 7900HT Fast Real-Time PCR System (Applied Biosystems, Foster City, CA).

Once mRNA is isolated, the first step in gene expression profiling by RT- PCR is the reverse transcription of the mRNA template into cDNA, which is then exponentially amplified in a PCR reaction. Two commonly used reverse transcriptases are avilo myeloblastosis virus reverse transcriptase (AMV-RT) and Moloney murine leukemia virus reverse transcriptase (MMLV-RT). The reverse transcription reaction typically is primed with specific primers, random hexamers, or oligo(dT) primers. The resulting cDNA product can be used as a template in the subsequent polymerase chain reaction.

The PCR step is carried out using a thermostable DNA-dependent DNA polymerase. The polymerase most commonly used in PCR systems is a Thermus aquaticus (Taq) polymerase. The selectivity of PCR results from the use of primers that are complementary to the DNA region targeted for amplification, i.e., regions of the cDNAs reverse transcribed from genes encoding macrophage marker proteins, such as CD68. Therefore, when qRT-PCR is employed in the present disclosure primers specific to each marker gene are based on the cDNA sequence of the gene. Commercial technologies such as SYBR$^{(R)}$ green or TaqMan$^{(R)}$ (Applied Biosystems, Foster City, CA) can be used in accordance with the vendor's instructions. Messenger RNA levels can be normalized for differences in loading among samples by comparing the levels of housekeeping genes such as beta-actin or GAPDH. The level of mRNA expression can be expressed relative to any single control sample such as mRNA from normal, non-tumor tissue or cells. Alternatively, it can be expressed relative to mRNA from a pool of tumor samples, or tumor cell lines, or from a commercially available set of control mRNA.

Suitable primer sets for PCR analysis of expression levels of genes encoding macrophage marker proteins such as CD68 can be designed and synthesized by one of skill in the art, without undue experimentation. Alternatively, PCR primer sets for practicing the present disclosure can be purchased from commercial sources, e.g., Applied Biosystems. PCR primers preferably are about 17 to 25 nucleotides in length. Primers can be designed to have a particular melting temperature (Tm), using conventional algorithms for Tm estimation. Software for primer design and Tm estimation are available commercially, e.g., Primer Express™ (Applied Biosystems), and also are available on the internet, e.g., Primer3 (Massachusetts Institute of Technology). By applying established principles of PCR primer design, a large number of different primers can be used to measure the expression level of any given gene, including macrophage marker genes such as CD14, CD68, MSR1, CSFR1, CD163 and CCR2.

In some embodiments of the disclosure, RNA analysis is performed using a technology that does not involve RNA extraction or isolation. One such technology is quantitative nuclease protection assay, which is commercially available under the name qNPA™ (High Throughput Genomics, Inc., Tucson, AZ). This technology can be advantageous when the tumor tissue samples to be analyzed are in the form of FFPE material. See, e.g., Roberts et al., 2007, Laboratory Investigation 87:979-997.

Protein analysis

In methods of the disclosure, macrophage marker gene expression such as CD68 can be detected at the protein level. Examples of methods for measuring the level of macrophage marker gene expression at the protein level include enzyme linked immunosorbent assay (ELISA) and IHC analysis.

ELISA

Performing a macrophage marker protein ELISA, e.g., CD68 ELISA, requires at least one antibody against a macrophage marker protein, i.e., the detection antibody. In an exemplary embodiment, CD68 is the macrophage marker protein. CD68 protein from a sample to be analyzed is immobilized on a solid support such as a polystyrene microtiter plate. This immobilization can be by non-specific binding of the CD68, e.g., through adsorption to the surface. Alternatively,

immobilization can be by specific binding, e.g., through binding of CD68 protein from the sample by a capture antibody (anti-CD68 antibody different from the detection antibody), in a "sandwich" ELISA. After the CD68 is immobilized, the detection antibody is added, and the detection antibody forms a complex with the bound CD68. The detection antibody is linked to an enzyme, either directly or indirectly, e.g., through a secondary antibody that specifically recognizes the detection antibody. Typically between each step, the plate, with bound CD68, is washed with a mild detergent solution. Typical ELISA protocols also include one or more blocking steps, which involve use of a non-specifically binding protein such as bovine serum albumin to block unwanted non-specific binding of protein reagents to the plate. After a final wash step, the plate is developed by addition of an appropriate enzyme substrate, to produce a visible signal, which indicates the quantity of CD68 in the sample. The substrate can be, e.g., a chromogenic substrate or a fluorogenic substrate. ELISA methods, reagents and equipment are well-known in the art and commercially available.

It is understood that the expression levels of other macrophage marker proteins, e.g., CCR2, CD14, CD163, CSF1R, and MSR1, as well as other macrophage specific marker proteins can be measured by ELISA using detecting antibodies specific for each macrophage marker protein.

Immunohistochemistry (IHC)

The number of macrophages in a given cell population can be determined (e.g., visualized) by immunohistochemistry. In addition, the percentage and density of cells in a sample that are positive for a given biomarker protein, such as CD68, can be determined by immunochemistry. Assaying a macrophage marker protein by IHC, e.g., CD68 IHC, requires at least one antibody against a macrophage marker protein, e.g., at least one anti-CD68 antibody. Numerous anti-CD68 antibodies suitable for IHC are commercially available. For example, suitable antibodies can be purchased from Dako North America, Inc. (Carpinteria, CA), abeam (Cambridge, MA), Abnova (Walnut, CA), R and D Systems (Minneapolis, MN) or Invitrogen (Carlsbad, CA). Using standard techniques, the anti-CD68 antibody can be used to detect the presence of CD68 protein in sections, e.g., 5 micron sections, obtained from tumors, including paraffin-embedded and frozen tumor sections. Typically, the tumor sections are initially treated in such a way as to retrieve the antigenic structure of proteins that were fixed in the initial process of collecting and preserving the tumor material. Slides are then blocked to prevent non-specific binding by the anti-CD68 detection antibody. The presence of CD68 protein is then detected by binding of the anti-CD68 antibody to the CD68 protein. The detection (primary) antibody is linked to an enzyme, either directly or indirectly, e.g., through a secondary antibody or polymer that specifically recognizes the detection (primary) antibody. Typically, the tumor sections are washed and blocked with nonspecific protein such as bovine serum albumin between steps. The slide is developed using an appropriate enzyme substrate to produce a visible signal. The samples can be counterstained with hematoxylin.

It is understood that the expression of other macrophage marker proteins, e.g., CCR2, CD14, CD163, CSF1R, and MSR1, as well as other macrophage specific marker proteins can be detected by IHC in a similar manner using antibodies specific for each macrophage marker protein.

Data Interpretation

A macrophage score for a tumor can be interpreted with respect to a threshold score. A macrophage score, or the expression level of a particular biomarker such as CD68, that is equal to or higher than the threshold score can be interpreted as predictive of the tumor being likely to be sensitive (responsive) to treatment with a VEGFR inhibitor, such as with axitinib. Alternatively, macrophage scores, or the expression level of a particular biomarker such as CD68, equal to or lower than the threshold score can be interpreted as predictive of a tumor being likely to be resistant (non-responsive) to treatment with a VEGFR inhibitor, such as axitinib.

An optimum threshold macrophage score, or CD68 expression level, can be determined (or at least approximated) empirically by performing a threshold determination analysis. Preferably, threshold determination analysis includes receiver operator characteristic (ROC) curve analysis. ROC curve analysis is an established statistical technique, the application of which is within ordinary skill in the art. For a discussion of ROC curve analysis, see generally Zweig et al., 1993, "Receiver operating characteristic (ROC) plots: a fundamental evaluation tool in clinical medicine," Clin. Chem. 39:561-577; and Pepe, 2003, The statistical evaluation of medical tests or classification and prediction, Oxford Press, New York.

Macrophage scores, CD68 expression levels, and the optimum threshold scores may vary from tumor type to tumor type. Therefore, a threshold determination analysis preferably is performed on one or more datasets representing any given tumor type to be tested using the present disclosure. The dataset used for threshold determination analysis includes: (a) actual response data (response or non-response), and (b) a macrophage score or CD68 expression level for each tumor sample from a group of tumors. Once a macrophage score or CD68 expression level threshold is determined with respect to a given tumor type, that threshold can be applied to interpret macrophage scores or CD68 expression levels from tumors of that tumor type.

The ROC curve analysis can be performed as follows. Any sample with a macrophage score or CD68 expression level greater than or equal to the threshold is identified as a responder (sensitive). Alternatively, any sample with a macrophage score or CD68 expression level less than or equal to the threshold is identified as a non-responder (resistant). For every macrophage score or CD68 expression level from a tested set of samples, "responders" and "non-responders" (hypothetical calls) are classified using that score as the threshold. This process enables calculation of TPR (y vector) and FPR (x vector) for each potential threshold, through comparison of hypothetical calls against the actual response data for the data set. Then an ROC curve is constructed by making a dot plot, using the TPR vector, and FPR vector. If the ROC curve is above the diagonal from (0, 0) point to (1.0, 0.5) point, it shows that the macrophage test result is a better test than random.

The ROC curve can be used to identify the best operating point. The best operating point is the one that yields the best balance between the cost of false positives weighed against the cost of false negatives. These costs need not be equal. The average expected cost of classification at point x,y in the ROC space is determined by the following formula.

$$C = (1\text{-}p)\ alpha^*x + p^*beta(l\text{-}y)$$

wherein:

alpha = cost of a false positive,
beta = cost of missing a positive (false negative), and
p = proportion of positive cases.

False positives and false negatives can be weighted differently by assigning different values for alpha and beta. For example, if it is decided to include more patients in the responder group at the cost of treating more patients who are non-responders, one can put more weight on alpha. In this case, it is assumed that the cost of false positive and false negative is the same (alpha equals to beta). Therefore, the average expected cost of classification at point x,y in the ROC space is:

$$C' = (l\text{-}p)^*x + p^*(l\text{-}y).$$

The smallest C' can be calculated after using all pairs of false positive and false negative (x, y). The optimum score threshold is calculated as the score of the (x, y) at C'.

In addition to predicting whether a tumor will be sensitive or resistant to a VEGFR inhibitor, such as axitinib, a macrophage score or CD68 expression level provides an approximate, but useful, indication of how likely a tumor is to be sensitive or resistant.

Test Kits

The disclosure includes a diagnostic test kit comprising certain components for performing methods of the present disclosure. A diagnostic test kit enhances convenience, speed and reproducibility in the performance of diagnostic assays. For example, in an exemplary qRT-PCR-based embodiment of the disclosure, a basic diagnostic test kit includes PCR primers for analyzing expression of macrophage markers, e.g., CD68. In other embodiments, a more elaborate test kit contains not only PCR primers, but also buffers, reagents and detailed instructions for measuring CD68 expression levels, using PCR technology. In some embodiments, the kit includes a test protocol and all the consumable components needed for the test, except the RNA sample(s).

In an exemplary DNA microarray-based embodiment of the disclosure, a test kit includes a microfluidic card (array) designed for use with a particular instrument. Optionally, the microfluidic card is a custom made device designed specifically for measurement of macrophage marker gene expression. Such custom microfluidic cards are commercially available. For example, the TaqMan Array is a 384-well microfluidic card (array) designed for use with the Applied Biosystems 7900HT Fast Real Time PCR System (Applied Biosystems, Foster City, CA). An exemplary fluidic card may include any combination of probes for measuring CCR2, CD14, CD68, CD163, CSF1R and/or MSR1 expression plus necessary controls or standards, e.g., for data normalization. Other macrophage marker proteins can also be included on a fluidic card for practicing the disclosure.

In some embodiments of the disclosure, the test kit contains materials for determining tumor macrophage content by IHC. An IHC kit, for example, may contain a primary antibody against a human macrophage marker, e.g., a mouse anti-human CD68 antibody, and a secondary antibody conjugated to a reporter enzyme, e.g., horseradish peroxidase. In some embodiments, the secondary antibody is replaced with a conjugated polymer that specifically recognizes

the primary antibody.

## EXAMPLES

The present disclosure is further illustrated by the following examples.

Example 1 - Percent and Density of CD3 and CD68 Positive Cells by Slides versus Blocks

This study was a 2-arm, randomized, open-label, multi-center phase 3 study of axitinib versus sorafenib in patients with metastatic renal cell carcinoma (mRCC), after failure following one prior systematic first-line regimen containing one or more of the following agents: sunitinib, bevacizumab + IFN $\alpha$, temsirolimus, or cytokine(s). Overall, 723 patients with mRCC were randomized and enrolled in this study, among which 52 axitinib-treated patients were evaluable for immunohistochemistry (IHC) analysis; further, 33 of the 52 patients were previously treated with Sutent®.

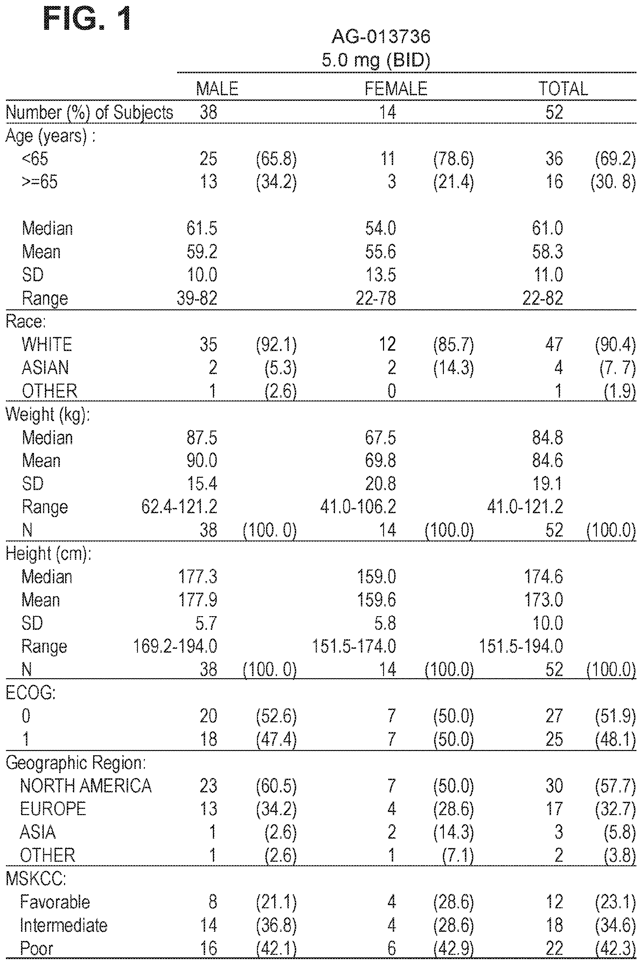

As shown in Figure 1, the majority of patients included in IHC analysis were white (90.4% of patients), male (69.2% of patients) and from North America (57.7% of patients) and Europe (32.7%). Overall mean (standard deviation) age, height and weight was 58.3 (11.0) years, 173.0 (10.0) cm, and 84.6 (19.1) kg, respectively. All patients had an ECOG performance status of 0 (51.9% of patients) or 1 (48.1% of patients). Overall, 23.1% of patients classified as favorable, and 34.6% and 42.3% of patients as intermediate and poor, respectively, for the Memorial Sloan-Kettering Cancer Center (MSKCC) prognostic group factors model for survival. In addition, MSKCC risk groups were derived using the following four risk factors: high lactate dehydrogenase (>1.5 x upper limit of normal), low serum hemoglobin (less than the lower limit of normal), high corrected serum calcium (>10 mg/dL), and absence of prior nephrectomy.

All 52 patients were evaluable for both CD3 and CD68. Of the 52 CD3 and CD68 evaluable patients, 26 donated formalin-fixed paraffin embedded (FFPE) tumor blocks, and 26 donated slides for analysis. Mosaic Laboratories provided FFPE material representing human normal and human cancer. Specimens were procured under an IRB-reviewed protocol (MOS001) that allows for use of remnant, de-identified, or anonymized human samples for *in vitro* analysis under the guidelines defining 'Exemption from Human Subject Research' as defined by the Office of Human Research Protection. The CD68 mouse monoclonal KP1 antibody (Catalog# M0814, Lot# 46406, Expiration date: Sep 2011) was purchased from Dako (Carpinteria, California, United States) and stored at 2-8°C in accordance with accompanying documentation. The mouse IgG isotype control antibody (Lot# 37211, Expiration date: Jul 2010) was purchased from Dako and stored at 2-8°C in accordance with accompanying documentation. IHC was performed in accordance with Mosaic Laboratories' SOPs. The CD68 IHC assay was designed and validated to be compatible with CLIA guidelines for "homebrew" class I test validation.

Staining was evaluated by a pathologist and evaluation of reactivity involved a combination of the following: cellular localization of CD68 staining; staining intensity; subcellular localization; and percentage of cells staining in the primary component of the tissue type of interest. Photomicrographs (20x magnification) were acquired with a Spot Insight QE Model 4.2 cooled charge-coupled device camera (Diagnostic Instruments, Sterling Heights, Michigan, United States) attached to a Nikon Eclipse 50i microscope.

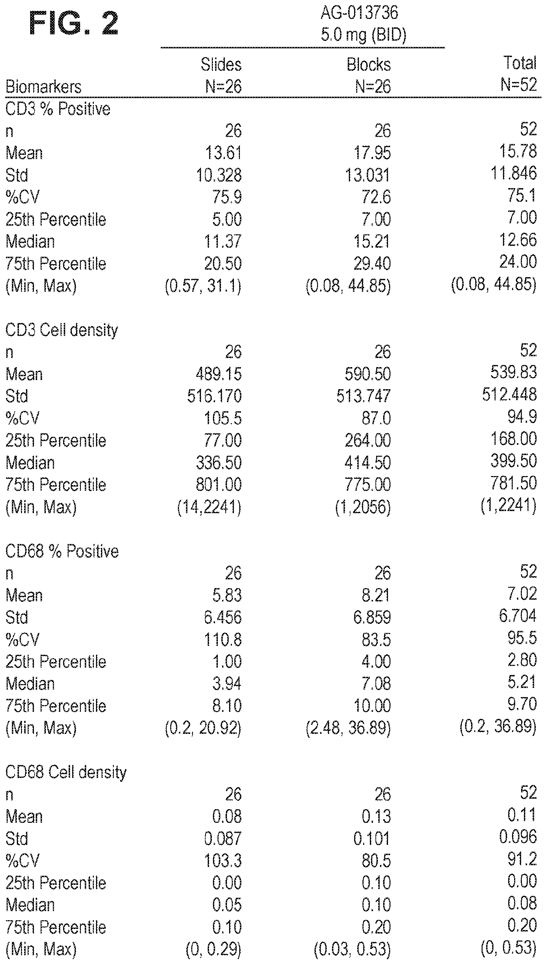

The mean percentage of CD3 and CD68 positive cells was slightly lower in slides than in blocks; 13.61% versus 17.95% for CD3 and 5.83% versus 8.21% for CD68. Similarly, a slightly lower mean cell density was observed in slides than in blocks; 489.15 cells/$mm^2$ versus 590.50 cells/$mm^2$ for CD3, and 0.08 cells/$mm^2$ versus 0.13 cells/$mm^2$ for CD68 (Figure 2).

Example 2 - Higher CD68 Expression Levels Correlate Positively with Favorable ORR and PFS, but Not OS

For IHC biomarker analysis, the Biomarker Analysis Set was used, which included all patients who received at least one dose of study treatment. The following efficacy endpoints were analyzed: PFS, OS, and objective response rate (ORR). Summary statistics were provided for percent and density of positive cells by response category (complete response [CR]+partial response [PR] versus stable disease [SD]+progressive disease [PD]) for each marker). The Wilcoxon Rank Sum test was performed to test for difference between response categories. Fisher's exact test was used to test for association between response category and biomarker stratum using median value as cut off point. Distribution of OS and PFS was compared between the biomarker stratum by biomarker median value as cut off point using the Kaplan-Meier method; p-values were not to be displayed when N<10 in either stratum. The estimated hazard ratio (HR) and its 2-sided 95% confidence intervals (CIs), and median event time and its 2-sided 95% CI were reported. Best response percent change in tumor volume was compared between the biomarker strata with median value as cut off point using Wilcoxon Rank Sum test.

For significant test results (p<0.05) in the ORR, PFS, and OS analysis between biomarker stratum, the receiver operating characteristics (ROC) curve was generated to further assess the potential for utility as patient selection markers. ROC analysis was performed on baseline CD68 values as continuous diagnostic markers in predicting binary patient

objective response (CR+PR vs. SD+PD). For time-dependent clinical outcomes PFS and OS, a time-dependent ROC, denoted as ROC(t), where t indicates time point of interest, was applied to analyze baseline CD68 values in predicting survival outcomes using the Kaplan-Meier estimator20. The optimal cut off point of the CD68 value for predicting clinical outcome was obtained from the point on the ROC curve having the minimum distance from the point with both sensitivity and specificity values of 1. The AUC was calculated using the trapezoidal rule.

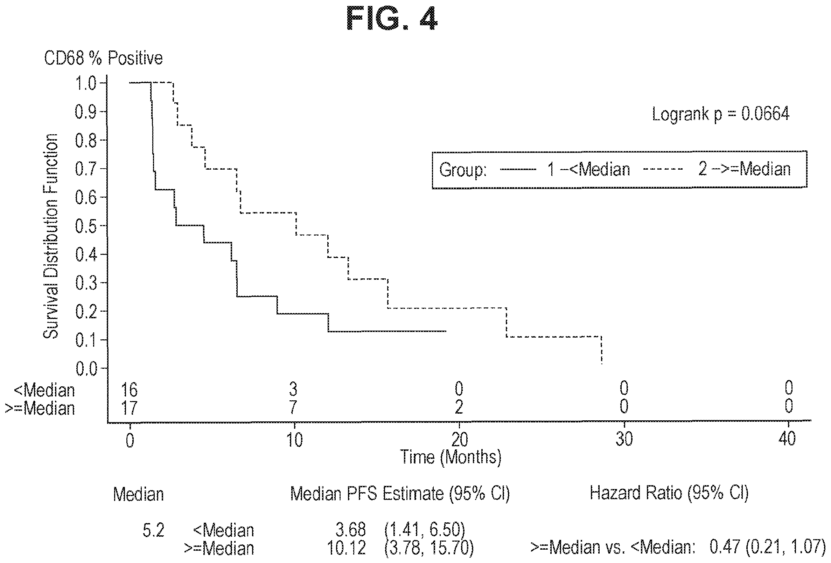

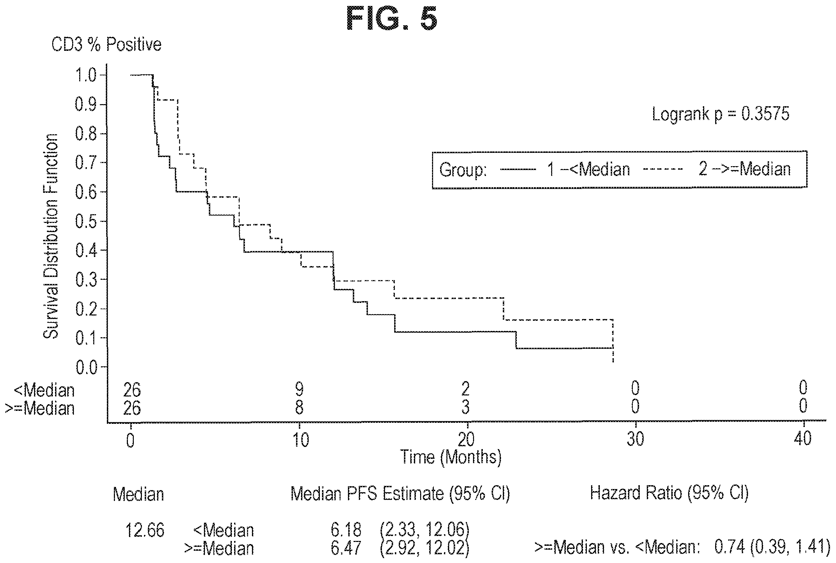

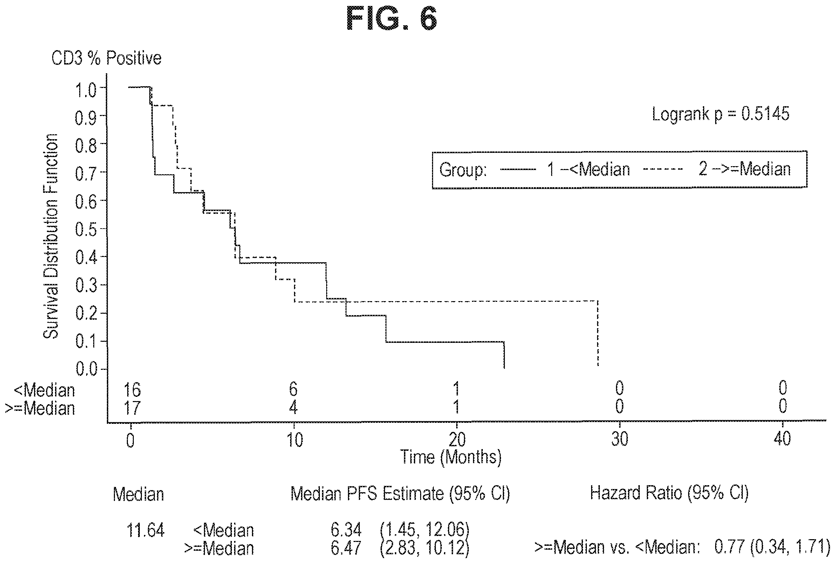

Higher CD68 expression associated with longer PFS and higher ORR was observed, however, no correlation was observed between CD68 expression and OS in patients. Regardless of prior treatment, median PFS in patients with ≥median CD68 values (cut off = 5.21% cells positive or 0.08 cells/$mm^2$) was 12.0 months for both cut off points vs 3.7 and 3.8 months respectively for patients with <median biomarker values (HR=0.42, logrank p-value≤ 0.01) when all patients were assessed regardless of prior treatment (Figure 3). However, for patients pre-treated with Sutent®, a similar but not statistically significant trend with PFS was observed (Figure 4). There were no statistically significant associations between CD3 levels and PFS, either regardless of prior treatment or for Sutent®-pre-treated patients (Figures 5 and 6).

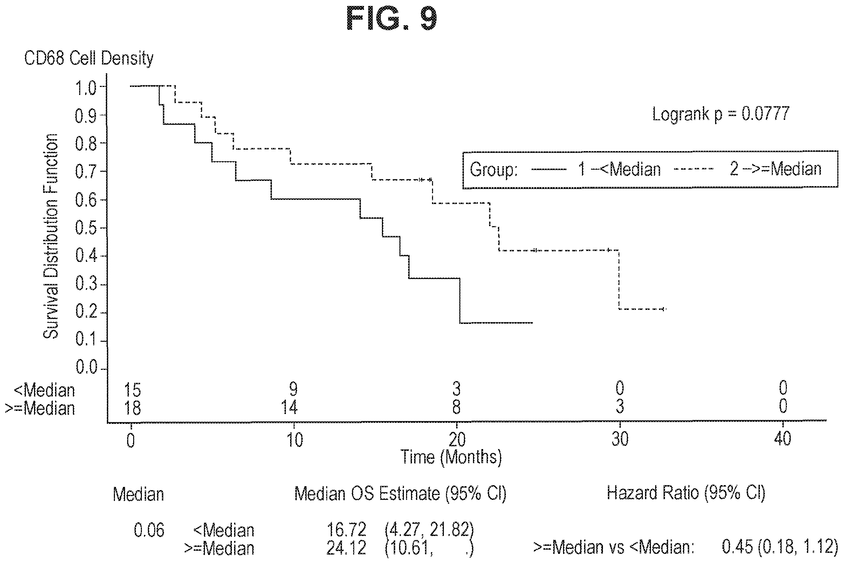

Further, regardless of prior treatment, median OS in patients with ≥median CD68 values (cut off = 5.21% cells positive or 0.08 cells/$mm^2$) was 20.0 months and 22.6 months versus 21.8 months or 17.8 months respectively for patients with <median biomarker values (HR>0.6 in all cases, not statistically significant) when all patients were assessed regardless of prior treatment (Figure 7). For patients pre-treated with Sutent® no statistically significant associations with OS were observed. There were no statistically significant associations between CD3 levels and OS either, regardless of treatment, or in Sutent®-pre-treated patients (Figure 8). Using ROC analysis, favorable OS was observed for patients with higher CD68 cell count, although the AUC value of 0.559 indicates low confidence in the predictive value of CD68 levels (Figure 9).

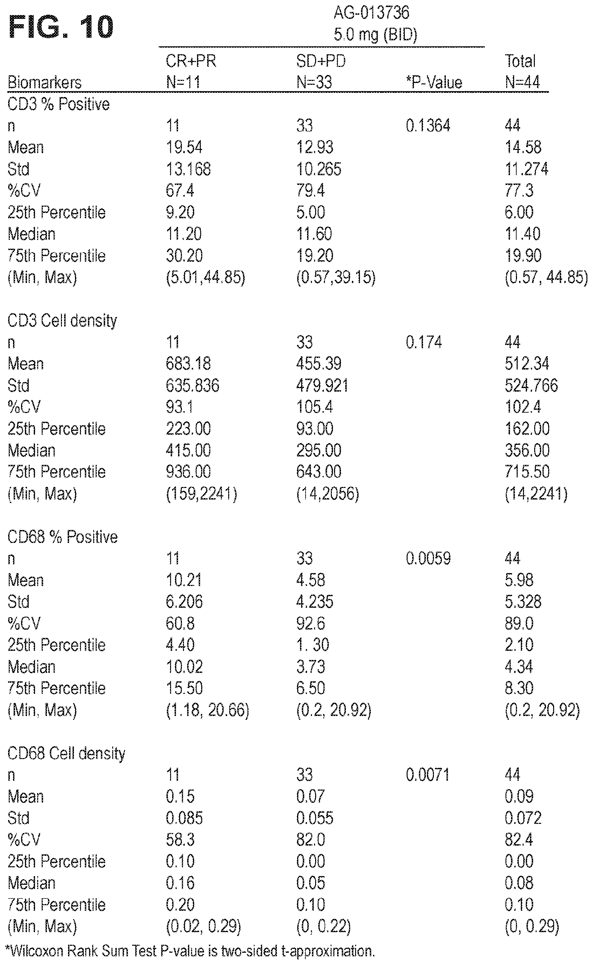

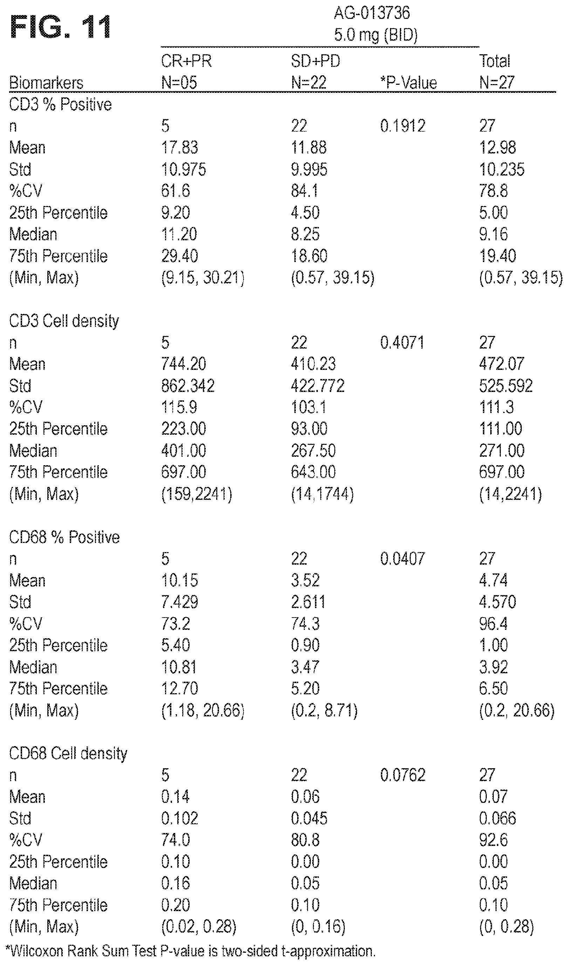

Lastly, regardless of prior treatment, CD68 levels measured by percentage positive cells (p-value=0.0059) or cell density (p-value=0.0071) were 2-fold higher in responders (CR+PR) versus non-responders (SD+PD) (Figure 10). For patients previously treated with Sutent®, CD68 levels measured by percentage positive cells (p-value=0.407) or cell density (p-value=0.0762) were 2-fold higher in responders versus non-responders (Figure 11). In ORR analysis for biomarker evaluable patients, patients with higher CD68 percent and density of positive cells tend to have better chance of tumor objective response. ROC analysis shows a predictive accuracy of 0.818 and 0.795 with CD68 percentage and density of positive cells, respectively. The AUCs of 0.791 and 0.784, indicate high overall predictive accuracy for ORR using CD68 biomarker. The optimal cutoff points for predicting ORR were 9.42% and 0.13 cells/$mm^2$ for percentage of CD68 positive cells and cell density, respectively.

Similar results were found in ORR analysis for biomarker evaluable patients with prior Sutent® treatment. Patients with higher percentage of CD68 positive cells and density tend to have better chance of tumor objective response. ROC analysis shows a predictive accuracy of 0.777 and 0.852 with percentage of CD68 positive cells and density, respectively. The AUCs of 0.809 and 0.764, indicate high overall diagnostic accuracy for ORR using CD68 biomarker. The optimal cutoff points for predicting ORR are 5.20% and 0.16 cells/$mm^2$ for percentage of CD68 positive cells and density, respectively.

## Claims

1. A method of identifying a tumor that is sensitive to treatment with axitinib, comprising: (a) measuring CD68 polypeptide expression level in a tissue sample from a tumor obtained from a human patient being considered for treatment with axitinib; and (b) comparing the CD68 expression level in step (a) against a threshold CD68 expression level determined by measuring CD68 polypeptide expression in tissue samples of tumors obtained from human patients previously treated with axitinib and shown to be resistant to axitinib and human patients previously treated with axitinib and shown to be sensitive to axitinib, wherein a CD68 expression level above the threshold level indicates that the tumor is sensitive to treatment with axitinib.

2. The method of claim 1, wherein the step of measuring CD68 polypeptide expression is performed by immunohistochemistry.

3. The method of claim 2, wherein the step of measuring CD68 polypeptide expression by immunohistochemistry is carried out by image analysis from a whole slide scan, where the percentage of CD68-positive cells in the sample is determined.

4. The method of claim 3, further comprising the step of determining the density of CD68-positive cells in the sample.

5. The method of any one of claims 1 to 4, wherein the tumor is selected from the group consisting of a breast tumor, a lung tumor, a kidney tumor, a colorectal tumor, and a pancreatic tumor, and is preferably a metastatic renal cell

cancer (mRCC) tumor.

6. Axitinib for use in the treatment of metastatic renal cell cancer (mRCC) by administration of axitinib to a patient determined to have a mRCC tumor that is sensitive to axitinib according to claim 5.

7. Axitinib for use in the treatment of cancer by (a) determination of the percentage of CD68-positive cells in a tumor from a subject; and (b) administration of axitinib to the subject if said percentage is at least 5%.

8. Axitinib for use in the treatment of cancer by (a) determination of the cell density of CD68-positive cells in a tumor from a subject; and (b) administration of axitinib to the subject if said cell density is at least 0.08 cells/$mm^2$.

9. Axitinib for use in the treatment of cancer by administration of axitinib to a subject with a tumor, wherein at least 2%, at least 3%, at least 4%, at least 4.5%, at least 4.6%, at least 4.7%, at least 4.8%, at least 4.9%, at least 5.0%, at least 5.1%, at least 5.2%, at least 5.3%, at least 5.4%, at least 5.5%, at least 5.6%, at least 5.7%, at least 5.8%, at least 5.9%, at least 6.0%, at least 6.5%, at least 7.0%, at least 8%, at least 9%, at least 10%, at least 15%, or at least 20% of the cells in said tumor are CD68-positive.

10. Axitinib for use in the treatment of cancer as claimed in claim 9 by administration of axitinib to a subject with a tumor, wherein at least 5% of the cells in said tumor are CD68-positive.

11. Axitinib for use in the treatment of cancer by administration of axitinib to a subject with a tumor, wherein the cell density of CD68-positive cells in said tumor is at least 0.05 cells/mm2, at least 0.06 cells/mm2, at least 0.07 cells/mm2, at least 0.08 cells/mm2, at least 0.09 cells/mm2, at least 1.0 cells/mm2, at least 1.1 cells/mm2, at least 1.2 cells/mm2, at least 1.3 cells/mm2, at least 1.4 cells/mm2, or at least 1.5 cells/mm2.

12. Axitinib for use in the treatment of cancer as claimed in claim 11 by administration of axitinib to a subject with a tumor, wherein the cell density of CD68-positive cells in said tumor is at least 0.08 cells/$mm^2$.

13. Axitinib for use in the treatment of cancer by (a) determination of the percentage of CD68-positive cells in a tumor from a subject; (b) determination of the cell density of CD68-positive cells in the tumor; and (c) administration of axitinib to the subject if said percentage is at least least 2%, at least 3%, at least 4%, at least 4.5%, at least 4.6%, at least 4.7%, at least 4.8%, at least 4.9%, at least 5.0%, at least 5.1%, at least 5.2%, at least 5.3%, at least 5.4%, at least 5.5%, at least 5.6%, at least 5.7%, at least 5.8%, at least 5.9%, at least 6.0%, at least 6.5%, at least 7.0%, at least 8%, at least 9%, at least 10%, at least 15%, or at least 20%; and said cell density is at least 0.05 cells/mm2, at least 0.06 cells/mm2, at least 0.07 cells/mm2, at least 0.08 cells/mm2, at least 0.09 cells/mm2, at least 1.0 cells/mm2, at least 1.1 cells/$mm^2$, at least 1.2 cells/mm2, at least 1.3 cells/mm2, at least 1.4 cells/mm2, or at least 1.5 cells/mm2.

14. Axitinib for use in the treatment of cancer as claimed in claim 13 by (a) determination of the percentage of CD68-positive cells in a tumor from a subject; (b) determination of the cell density of CD68-positive cells in the tumor; and (c) administration of axitinib to the subject if said percentage is at least 5% and said cell density is at least 0.08 cells/$mm^2$.

15. Axitinib for use in the treatment of cancer as claimed in any one of claims 7 to 14, wherein said tumor is selected from the group consisting of a breast tumor, a lung tumor, a kidney tumor, a colorectal tumor, and a pancreatic tumor, and is preferably a metastatic renal cell cancer (mRCC) tumor.

16. Axitinib for use in the treatment of cancer as claimed in claim 7, 8, 13 or 14, wherein the step of measuring the percentage or cell density of CD68 positive cells is performed using immunohistochemistry.

17. Axitinib for use in the treatment of cancer as claimed in claim 16, wherein the step of measuring the percentage or cell density of CD68 positive cells is performed using immunohistochemistry and the use of image analysis from a whole slide scan.

18. Use of a diagnostic test kit comprising certain components for performing methods of any one of claims 1 to 5, the components including, (a) for a quantitative reverse transcriptase polymerase chain reaction (qRT-PCR)-based kit, PCR primers for analyzing expression of the macrophage marker CD68 and optionally buffers, reagents and detailed instructions for measuring CD68 expression levels, using PCR technology; (b) for a DNA microarray-based kit, a

microfluidic card (array) designed for use with a particular instrument; or (c) materials for determining tumor macrophage content by IHC.

## Patentansprüche

1. Verfahren zum Identifizieren eines Tumors, der gegenüber einer Behandlung mit Axitinib empfindlich ist, umfassend: (a) Messen eines CD68-Polypeptidexpressionslevels in einer Gewebeprobe von einem Tumor, erhalten von einem menschlichen Patienten, der für eine Behandlung mit Axitinib in Betracht gezogen wird, und (b) Vergleichen des CD68-Expressionslevels in Schritt (a) gegenüber einem Schwellenwert-CD68-Expressionslevel, der durch Messen der CD68-Polypeptidexpression in Gewebeproben von Tumoren, erhalten von menschlichen Patienten, die zuvor mit Axitinib behandelt worden waren, und für die sich zeigte, dass sie gegenüber Axitinib resistent sind, und von menschlichen Patienten, die zuvor mit Axitinib behandelt worden waren und für die sich zeigte, dass sie gegenüber Axitinib empfindlich sind, wobei ein CD68-Expressionslevel über dem Schwellenwertlevel anzeigt, dass der Tumor gegenüber einer Behandlung mit Axitinib empfindlich ist.

2. Verfahren gemäß Anspruch 1, wobei der Schritt des Messens der CD68-Polypeptidexpression durch Immunhistochemie durchgeführt wird.

3. Verfahren gemäß Anspruch 2, wobei der Schritt des Messens der CD68-Polypeptidexpression durch Immunhistochemie durch Bildanalyse von einem Scan des gesamten Objektträgers ("whole slide scan") durchgeführt wird, wobei der Prozentanteil an CD68-positiven Zellen in der Probe bestimmt wird.

4. Verfahren gemäß Anspruch 3, das weiterhin den Schritt des Bestimmens der Dichte von CD68-positiven Zellen in der Probe umfasst.

5. Verfahren gemäß einem der Ansprüche 1 bis 4, wobei der Tumor ausgewählt ist aus der Gruppe, bestehend aus einem Brusttumor, einem Lungentumor, einem Nierentumor, einem kolorektalen Tumor und einem pankreatischen Tumor, und vorzugsweise ein metastasierender Nierenzellenkrebs (mRCC)-Tumor ist.

6. Axitinib zur Verwendung bei der Behandlung von metastasierendem Nierenzellenkrebs (mRCC) durch Verabreichung von Axitinib an einen Patienten, für den bestimmt wurde, dass er einen mRCC-Tumor hat, der gegenüber Axitinib empfindlich ist, gemäß Anspruch 5.

7. Axitinib zur Verwendung bei der Behandlung von Krebs durch (a) Bestimmung des Prozentanteils von CD68-positiven Zellen in einem Tumor von einem Subjekt und (b) Verabreichung von Axitinib an das Subjekt, wenn dieser Prozentanteil mindestens 5% ist.

8. Axitinib zur Verwendung bei der Behandlung von Krebs durch (a) Bestimmung der Zelldichte von CD68-positiven Zellen in einem Tumor von einem Subjekt und (b) Verabreichung von Axitinib an das Subjekt, wenn diese Zelldichte mindestens 0,08 Zellen/$mm^2$ ist.

9. Axitinib zur Verwendung bei der Behandlung von Krebs durch Verabreichung von Axitinib an ein Subjekt mit einem Tumor, wobei mindestens 2%, mindestens 3%, mindestens 4%, mindestens 4,5%, mindestens 4,6%, mindestens 4,7%, mindestens 4,8%, mindestens 4,9%, mindestens 5,0%, mindestens 5,1%, mindestens 5,2%, mindestens 5,3%, mindestens 5,4%, mindestens 5,5%, mindestens 5,6%, mindestens 5,7%, mindestens 5,8%, mindestens 5,9%, mindestens 6,0%, mindestens 6,5%, mindestens 7,0%, mindestens 8%, mindestens 9%, mindestens 10%, mindestens 15% oder mindestens 20% der Zellen in dem Tumor CD68-positiv sind.

10. Axitinib zur Verwendung bei der Behandlung von Krebs, wie in Anspruch 9 beansprucht, durch Verabreichung von Axitinib an ein Subjekt mit einem Tumor, wobei mindestens 5% der Zellen in dem Tumor CD68-positiv sind.

11. Axitinib zur Verwendung bei der Behandlung von Krebs durch Verabreichung von Axitinib an ein Subjekt mit einem Tumor, wobei die Zelldichte von CD68-positiven Zellen in dem Tumor mindestens 0,05 Zellen/$mm^2$, mindestens 0,06 Zellen/$mm^2$, mindestens 0,07 Zellen/$mm^2$, mindestens 0,08 Zellen/$mm^2$, mindestens 0,09 Zellen/$mm^2$, mindestens 1,0 Zellen/$mm^2$, mindestens 1,1 Zellen/$mm^2$, mindestens 1,2 Zellen/$mm^2$, mindestens 1,3 Zellen/$mm^2$, mindestens 1,4 Zellen/$mm^2$ oder mindestens 1,5 Zellen/$mm^2$ ist.

12. Axitinib zur Verwendung bei der Behandlung von Krebs, wie in Anspruch 11 beansprucht, durch Verabreichung von Axitinib an ein Subjekt mit einem Tumor, wobei die Zelldichte von CD68-positiven Zellen in dem Tumor mindestens 0,08 Zellen/$mm^2$ ist.

13. Axitinib zur Verwendung bei der Behandlung von Krebs durch (a) Bestimmung des Prozentanteils von CD68-positiven Zellen in einem Tumor von einem Subjekt, (b) Bestimmung der Zelldichte von CD68-positiven Zellen in dem Tumor und (c) Verabreichung von Axitinib an das Subjekt, wenn dieser Prozentanteil mindestens 2%, mindestens 3%, mindestens 4%, mindestens 4,5%, mindestens 4,6%, mindestens 4,7%, mindestens 4,8%, mindestens 4,9%, mindestens 5,0%, mindestens 5,1%, mindestens 5,2%, mindestens 5,3%, mindestens 5,4%, mindestens 5,5%, mindestens 5,6%, mindestens 5,7%, mindestens 5,8%, mindestens 5,9%, mindestens 6,0%, mindestens 6,5%, mindestens 7,0%, mindestens 8%, mindestens 9%, mindestens 10%, mindestens 15% oder mindestens 20% ist und diese Zelldichte mindestens 0,05 Zellen/$mm^2$, mindestens 0,06 Zellen/$mm^2$, mindestens 0,07 Zellen/$mm^2$, mindestens 0,08 Zellen/$mm^2$, mindestens 0,09 Zellen/$mm^2$, mindestens 1,0 Zellen/$mm^2$, mindestens 1,1 Zellen/$mm^2$, mindestens 1,2 Zellen/$mm^2$, mindestens 1,3 Zellen/$mm^2$, mindestens 1,4 Zellen/$mm^2$ oder mindestens 1,5 Zellen/$mm^2$ ist.

14. Axitinib zur Verwendung bei der Behandlung von Krebs, wie in Anspruch 13 beansprucht, durch (a) Bestimmung des Prozentanteils von CD68-positiven Zellen in einem Tumor von einem Subjekt, (b) Bestimmung der Zelldichte von CD68-positiven Zellen in dem Tumor und (c) Verabreichung von Axitinib an das Subjekt, wenn dieser Prozentanteil mindestens 5% ist und diese Zelldichte mindestens 0,08 Zellen/$mm^2$ ist.

15. Axitinib zur Verwendung bei der Behandlung von Krebs, wie in einem der Ansprüche 7 bis 14 beansprucht, wobei der Tumor ausgewählt ist aus der Gruppe, bestehend aus einem Brusttumor, einem Lungentumor, einem Nierentumor, einem kolorektalen Tumor und einem pankreatischen Tumor, und vorzugsweise ein metastasierender Nierenzellenkrebs (mRCC)-Tumor ist.

16. Axitinib zur Verwendung bei der Behandlung von Krebs, wie in Anspruch 7, 8, 13 oder 14 beansprucht, wobei der Schritt des Messens des Prozentanteils oder der Zelldichte von CD68-positiven Zellen unter Verwendung von Immunhistochemie durchgeführt wird.

17. Axitinib zur Verwendung bei der Behandlung von Krebs, wie in Anspruch 16 beansprucht, wobei der Schritt des Messens des Prozentanteils oder der Zelldichte von CD68-positiven Zellen unter Verwendung von Immunhistochemie und Verwendung von Bildanalyse von einem Scan des gesamten Objektträgers ("whole slide scan") durchgeführt wird.

18. Verwendung eines diagnostischen Testkits, umfassend bestimmte Komponenten zum Durchführen der Verfahren gemäß einem der Ansprüche 1 bis 5, wobei die Komponenten umfassen: für einen Kit, der auf einer quantitativen reverse Transkriptase-Polymerase-Kettenreaktion (qRT-PCR) basiert, PCR-Primer zum Analysieren der Expression des Makrophagenmarkers CD68 und gegebenenfalls Puffer, Reagentien und detaillierte Anweisungen zum Messen von CD68-Expressionsleveln unter Verwendung von PCR-Technologie, (b) für einen Kit, der auf einem DNA-Mikroarray basiert, eine Mikrofluidic-Karte (einen Mikrofluidic-Array), die/der für die Verwendung mit einem bestimmten Instrument entworfen wurde, oder (c) Materialien zur Bestimmung des Tumor-Makrophagengehalts durch IHC.

## Revendications

1. Procédé d'identification d'une tumeur qui est sensible à un traitement avec de l'axitinib, comprenant : (a) la mesure du taux d'expression du polypeptide CD68 dans un échantillon tissulaire provenant d'une tumeur obtenue d'un patient humain considéré pour un traitement avec de l'axitinib ; et (b) la comparaison du taux d'expression du CD68 dans l'étape (a) contre un taux seuil d'expression du CD68 déterminé en mesurant l'expression du polypeptide CD68 dans des échantillons tissulaires de tumeurs obtenues de patients humains traités auparavant avec de l'axitinib et démontrés comme étant résistants à l'axitinib et de patients humains traités auparavant avec de l'axitinib et démontrés comme étant sensibles à l'axitinib, dans lequel un taux d'expression du CD68 supérieur au taux seuil indique que la tumeur est sensible à un traitement avec de l'axitinib.

2. Procédé selon la revendication 1, dans lequel l'étape de mesure de l'expression du polypeptide CD68 est effectuée par immunohistochimie.

3. Procédé selon la revendication 2, dans lequel l'étape de mesure de l'expression du polypeptide CD68 par immunohistochimie est réalisée par analyse d'images à partir d'un balayage de lames entières, où le pourcentage de cellules CD68-positives dans l'échantillon est déterminé.

4. Procédé selon la revendication 3, comprenant en outre l'étape de détermination de la densité des cellules CD68-positives dans l'échantillon.

5. Procédé selon l'une quelconque des revendications 1 à 4, dans lequel la tumeur est choisie dans le groupe constitué d'une tumeur du sein, une tumeur du poumon, une tumeur du rein, une tumeur colo-rectale, et une tumeur pancréatique, et est de préférence une tumeur de cancer à cellules rénales métastasique (mRCC).

6. Axitinib pour une utilisation dans le traitement du cancer à cellules rénales métastasique (mRCC) par l'administration d'axitinib à un patient déterminé comme ayant une tumeur de mRCC qui est sensible à l'axitinib selon la revendication 5.

7. Axitinib pour une utilisation dans le traitement du cancer par (a) la détermination du pourcentage de cellules CD68-positives dans une tumeur provenant d'un sujet ; et (b) l'administration d'axitinib au sujet si ledit pourcentage est d'au moins 5 %.

8. Axitinib pour une utilisation dans le traitement du cancer par (a) la détermination de la densité cellulaire des cellules CD68-positives dans une tumeur provenant d'un sujet ; et (b) l'administration d'axitinib au sujet si ladite densité cellulaire est d'au moins 0,08 cellule/$mm^2$.

9. Axitinib pour une utilisation dans le traitement du cancer par l'administration d'axitinib à un sujet ayant une tumeur, dans lequel au moins 2 %, au moins 3 %, au moins 4 %, au moins 4,5 %, au moins 4,6 %, au moins 4,7 %, au moins 4, 8 %, au moins 4,9 %, au moins 5,0 %, au moins 5,1 %, au moins 5,2 %, au moins 5,3 %, au moins 5,4 %, au moins 5,5 %, au moins 5,6 %, au moins 5,7 %, au moins 5,8 %, au moins 5,9 %, au moins 6,0 %, au moins 6,5 %, au moins 7,0 %, au moins 8 %, au moins 9 %, au moins 10 %, au moins 15 %, ou au moins 20 % des cellules dans ladite tumeur sont CD68-positives.

10. Axitinib pour une utilisation dans le traitement du cancer selon la revendication 9 par l'administration d'axitinib à un sujet ayant une tumeur, dans lequel au moins 5 % des cellules dans ladite tumeur sont CD68-positives.

11. Axitinib pour une utilisation dans le traitement du cancer par l'administration d'axitinib à un sujet ayant une tumeur, dans lequel la densité cellulaire des cellules CD68-positives dans ladite tumeur est d'au moins 0,05 cellule/$mm^2$, au moins 0,06 cellule/$mm^2$, au moins 0,07 cellule/$mm^2$, au moins 0,08 cellule/$mm^2$, au moins 0,09 cellule/$mm^2$, au moins 1,0 cellule/$mm^2$, au moins 1,1 cellule/$mm^2$, au moins 1,2 cellule/$mm^2$, au moins 1,3 cellule/$mm^2$, au moins 1,4 cellule/$mm^2$, ou au moins 1,5 cellule/$mm^2$.

12. Axitinib pour une utilisation dans le traitement du cancer selon la revendication 11 par l'administration d'axitinib à un sujet ayant une tumeur, dans lequel la densité cellulaire des cellules CD68-positives dans ladite tumeur est d'au moins 0,08 cellule/$mm^2$.

13. Axitinib pour une utilisation dans le traitement du cancer par (a) la détermination du pourcentage de cellules CD68-positives dans une tumeur provenant d'un sujet ; (b) la détermination de la densité cellulaire des cellules CD68-positives dans la tumeur ; et (c) l'administration d'axitinib au sujet si ledit pourcentage est d'au moins 2 %, au moins 3 %, au moins 4 %, au moins 4,5 %, au moins 4,6 %, au moins 4,7 %, au moins 4,8 %, au moins 4,9 %, au moins 5,0 %, au moins 5,1 %, au moins 5,2 %, au moins 5,3 %, au moins 5,4 %, au moins 5,5 %, au moins 5,6 %, au moins 5,7 %, au moins 5,8 %, au moins 5,9 %, au moins 6,0 %, au moins 6,5 %, au moins 7,0 %, au moins 8 %, au moins 9 %, au moins 10 %, au moins 15 %, ou au moins 20 % ; et ladite densité cellulaire est d'au moins 0,05 cellule/$mm^2$, au moins 0,06 cellule/$mm^2$, au moins 0,07 cellule/$mm^2$, au moins 0,08 cellule/$mm^2$, au moins 0,09 cellule/$mm^2$, au moins 1,0 cellule/$mm^2$, au moins 1,1 cellule/$mm^2$, au moins 1,2 cellule/$mm^2$, au moins 1,3 cellule/$mm^2$, au moins 1,4 cellule/$mm^2$, ou au moins 1,5 cellule/$mm^2$.

14. Axitinib pour une utilisation dans le traitement du cancer selon la revendication 13 par (a) la détermination du pourcentage de cellules CD68-positives dans une tumeur provenant d'un sujet ; (b) la détermination de la densité cellulaire des cellules CD68-positives dans la tumeur ; et (c) l'administration d'axitinib au sujet si ledit pourcentage est d'au moins 5 % et ladite densité cellulaire est d'au moins 0,08 cellule/$mm^2$.

**15.** Axitinib pour une utilisation dans le traitement du cancer selon l'une quelconque des revendications 7 à 14, dans lequel ladite tumeur est choisie dans le groupe constitué d'une tumeur du sein, une tumeur du poumon, une tumeur du rein, une tumeur colorectale, et une tumeur pancréatique, et est de préférence une tumeur de cancer à cellules rénales métastasique (mRCC).

**16.** Axitinib pour une utilisation dans le traitement du cancer selon la revendication 7, 8, 13 ou 14, dans lequel l'étape de mesure du pourcentage ou de la densité cellulaire des cellules CD68-positives est effectuée en utilisant l'immunohistochimie.

**17.** Axitinib pour une utilisation dans le traitement du cancer selon la revendication 16, dans lequel l'étape de mesure du pourcentage ou de la densité cellulaire des cellules CD68-positives est effectuée en utilisant l'immunohistochimie et l'utilisation de l'analyse d'images à partir d'un balayage de lames entières.

**18.** Utilisation d'un kit de test de diagnostic comprenant certains composants pour effectuer des procédés selon l'une quelconque des revendications 1 à 5, les composants comprenant, (a) pour un kit basé sur une transcriptase inverse-réaction en chaîne de la polymérase quantitative (qRT-PCR), des amorces de PCR pour l'analyse de l'expression du marqueur des macrophages CD68 et éventuellement des tampons, des réactifs et des instructions détaillées pour mesurer les taux d'expression du CD68, en utilisant la technologie de la PCR ; (b) pour un kit basé sur des micropuces d'ADN, une carte (puce) microfluidique conçue pour une utilisation avec un instrument particulier ; ou (c) des matériaux pour déterminer la teneur en macrophages de la tumeur par IHC.

FIG. 1

| | AG-013736 5.0 mg (BID) | | | | | |
|---|---|---|---|---|---|---|
| | MALE | | FEMALE | | TOTAL | |
| Number (%) of Subjects | 38 | | 14 | | 52 | |
| Age (years) : | | | | | | |
| <65 | 25 | (65.8) | 11 | (78.6) | 36 | (69.2) |
| >=65 | 13 | (34.2) | 3 | (21.4) | 16 | (30. 8) |
| Median | 61.5 | | 54.0 | | 61.0 | |
| Mean | 59.2 | | 55.6 | | 58.3 | |
| SD | 10.0 | | 13.5 | | 11.0 | |
| Range | 39-82 | | 22-78 | | 22-82 | |
| Race: | | | | | | |
| WHITE | 35 | (92.1) | 12 | (85.7) | 47 | (90.4) |
| ASIAN | 2 | (5.3) | 2 | (14.3) | 4 | (7. 7) |
| OTHER | 1 | (2.6) | 0 | | 1 | (1.9) |
| Weight (kg): | | | | | | |
| Median | 87.5 | | 67.5 | | 84.8 | |
| Mean | 90.0 | | 69.8 | | 84.6 | |
| SD | 15.4 | | 20.8 | | 19.1 | |
| Range | 62.4-121.2 | | 41.0-106.2 | | 41.0-121.2 | |
| N | 38 | (100. 0) | 14 | (100.0) | 52 | (100.0) |
| Height (cm): | | | | | | |
| Median | 177.3 | | 159.0 | | 174.6 | |
| Mean | 177.9 | | 159.6 | | 173.0 | |
| SD | 5.7 | | 5.8 | | 10.0 | |
| Range | 169.2-194.0 | | 151.5-174.0 | | 151.5-194.0 | |
| N | 38 | (100. 0) | 14 | (100.0) | 52 | (100.0) |
| ECOG: | | | | | | |
| 0 | 20 | (52.6) | 7 | (50.0) | 27 | (51.9) |
| 1 | 18 | (47.4) | 7 | (50.0) | 25 | (48.1) |
| Geographic Region: | | | | | | |
| NORTH AMERICA | 23 | (60.5) | 7 | (50.0) | 30 | (57.7) |
| EUROPE | 13 | (34.2) | 4 | (28.6) | 17 | (32.7) |
| ASIA | 1 | (2.6) | 2 | (14.3) | 3 | (5.8) |
| OTHER | 1 | (2.6) | 1 | (7.1) | 2 | (3.8) |
| MSKCC: | | | | | | |
| Favorable | 8 | (21.1) | 4 | (28.6) | 12 | (23.1) |
| Intermediate | 14 | (36.8) | 4 | (28.6) | 18 | (34.6) |
| Poor | 16 | (42.1) | 6 | (42.9) | 22 | (42.3) |

FIG. 2

| Biomarkers | AG-013736 5.0 mg (BID) Slides N=26 | Blocks N=26 | Total N=52 |
|---|---|---|---|
| CD3 % Positive | | | |
| n | 26 | 26 | 52 |
| Mean | 13.61 | 17.95 | 15.78 |
| Std | 10.328 | 13.031 | 11.846 |
| %CV | 75.9 | 72.6 | 75.1 |
| 25th Percentile | 5.00 | 7.00 | 7.00 |
| Median | 11.37 | 15.21 | 12.66 |
| 75th Percentile | 20.50 | 29.40 | 24.00 |
| (Min, Max) | (0.57, 31.1) | (0.08, 44.85) | (0.08, 44.85) |
| CD3 Cell density | | | |
| n | 26 | 26 | 52 |
| Mean | 489.15 | 590.50 | 539.83 |
| Std | 516.170 | 513.747 | 512.448 |
| %CV | 105.5 | 87.0 | 94.9 |
| 25th Percentile | 77.00 | 264.00 | 168.00 |
| Median | 336.50 | 414.50 | 399.50 |
| 75th Percentile | 801.00 | 775.00 | 781.50 |
| (Min, Max) | (14,2241) | (1,2056) | (1,2241) |
| CD68 % Positive | | | |
| n | 26 | 26 | 52 |
| Mean | 5.83 | 8.21 | 7.02 |
| Std | 6.456 | 6.859 | 6.704 |
| %CV | 110.8 | 83.5 | 95.5 |
| 25th Percentile | 1.00 | 4.00 | 2.80 |
| Median | 3.94 | 7.08 | 5.21 |
| 75th Percentile | 8.10 | 10.00 | 9.70 |
| (Min, Max) | (0.2, 20.92) | (2.48, 36.89) | (0.2, 36.89) |
| CD68 Cell density | | | |
| n | 26 | 26 | 52 |
| Mean | 0.08 | 0.13 | 0.11 |
| Std | 0.087 | 0.101 | 0.096 |
| %CV | 103.3 | 80.5 | 91.2 |
| 25th Percentile | 0.00 | 0.10 | 0.00 |
| Median | 0.05 | 0.10 | 0.08 |
| 75th Percentile | 0.10 | 0.20 | 0.20 |
| (Min, Max) | (0, 0.29) | (0.03, 0.53) | (0, 0.53) |

FIG. 3

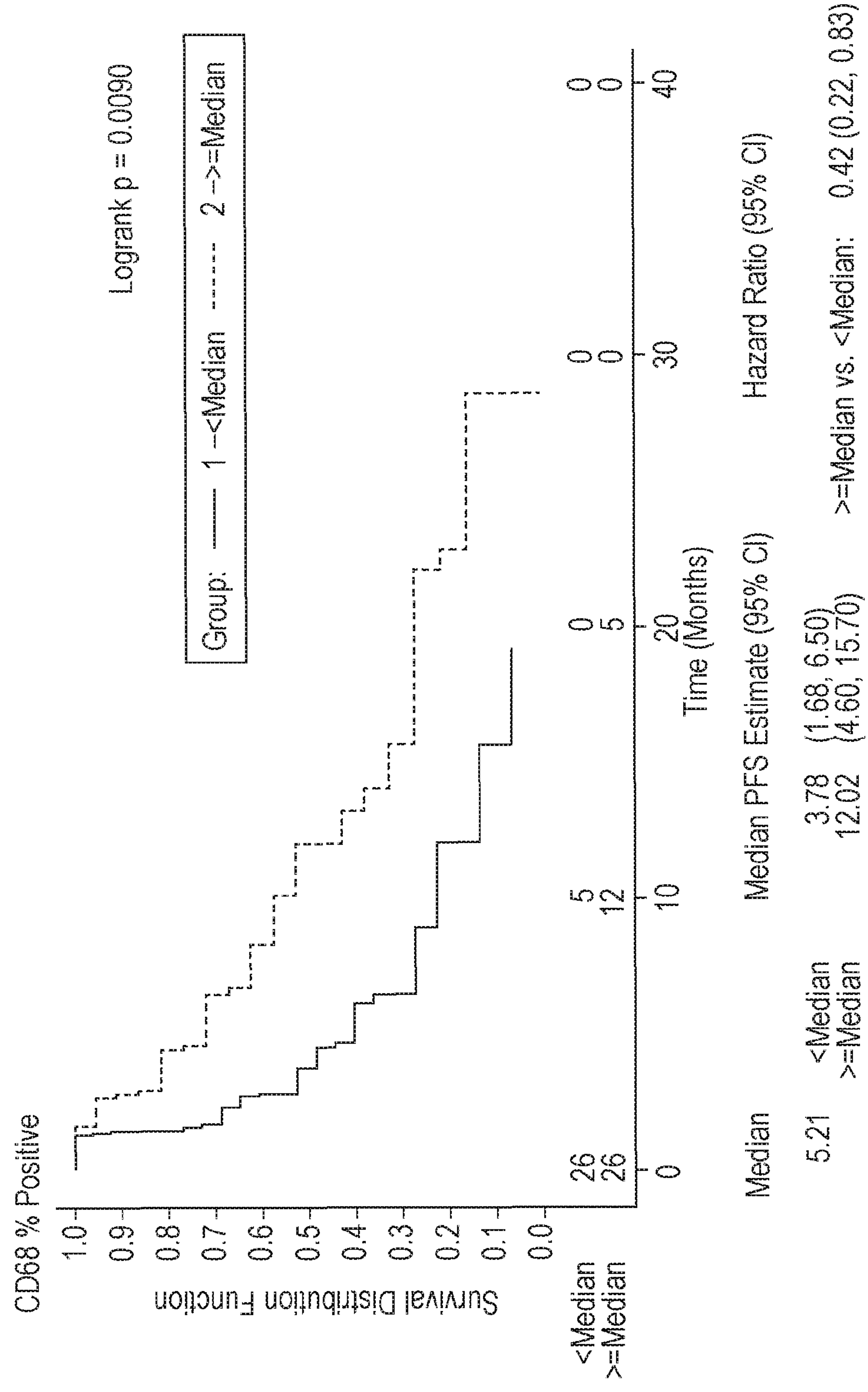
CD68 % Positive
Logrank p = 0.0090
Group: —— 1 –<Median ------ 2 –>=Median
Survival Distribution Function
1.0
0.9
0.8
0.7
0.6
0.5
0.4
0.3
0.2
0.1
0.0
0
10
20
30
40
Time (Months)
<Median 26 5 0 0 0
>=Median 26 12 5 0 0
Median 5.21
<Median
>=Median
Median PFS Estimate (95% CI)
3.78 (1.68, 6.50)
12.02 (4.60, 15.70)
Hazard Ratio (95% CI)
>=Median vs. <Median: 0.42 (0.22, 0.83)

# FIG. 4

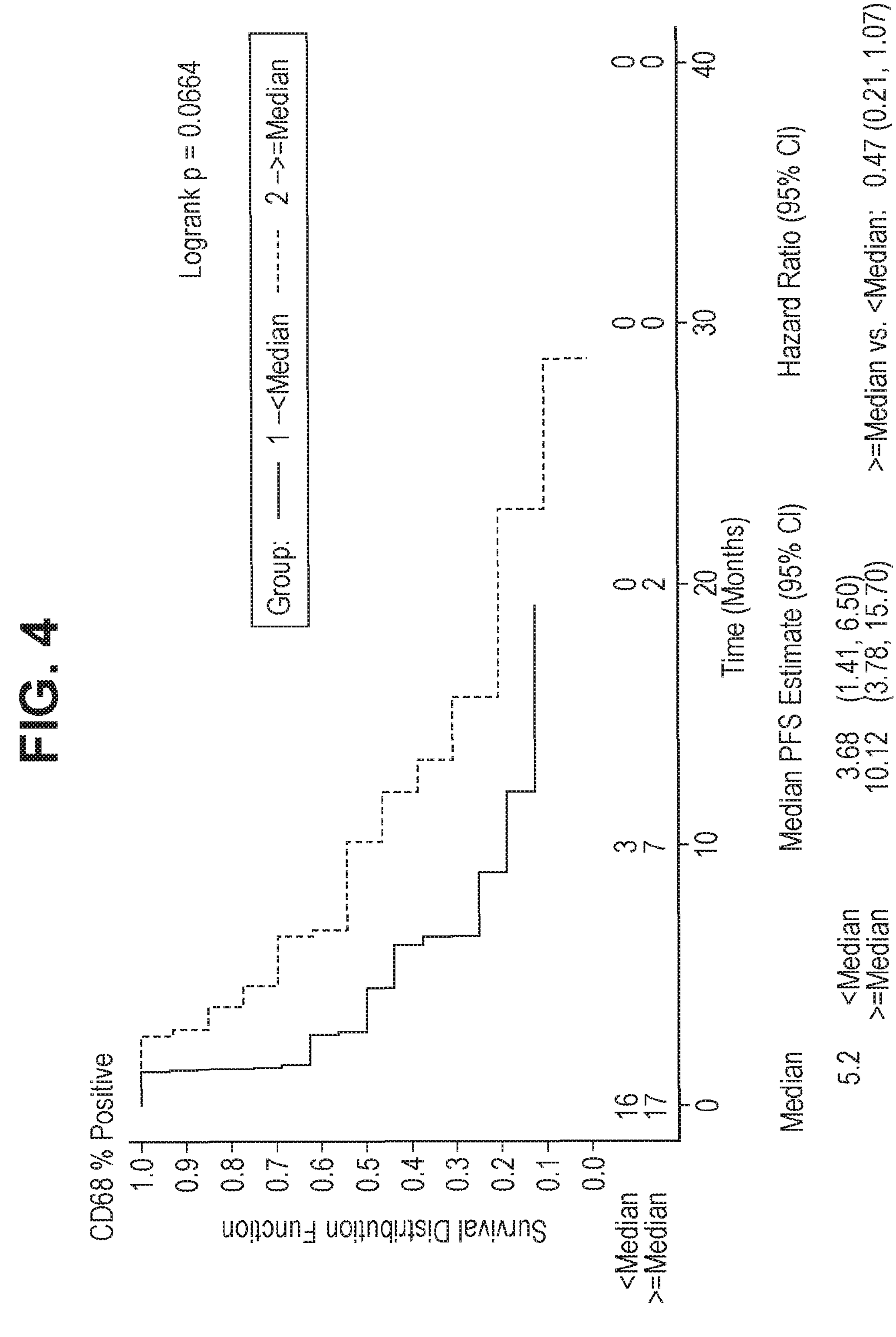
CD68 % Positive
Survival Distribution Function
1.0
0.9
0.8
0.7
0.6
0.5
0.4
0.3
0.2
0.1
0.0
0
10
20
30
40
Time (Months)
Logrank p = 0.0664
Group: 1 -<Median 2 ->=Median
<Median 16 3 0 0 0
>=Median 17 7 2 0 0
Median 5.2
Median PFS Estimate (95% CI)
<Median 3.68 (1.41, 6.50)
>=Median 10.12 (3.78, 15.70)
Hazard Ratio (95% CI)
>=Median vs. <Median: 0.47 (0.21, 1.07)

FIG. 5

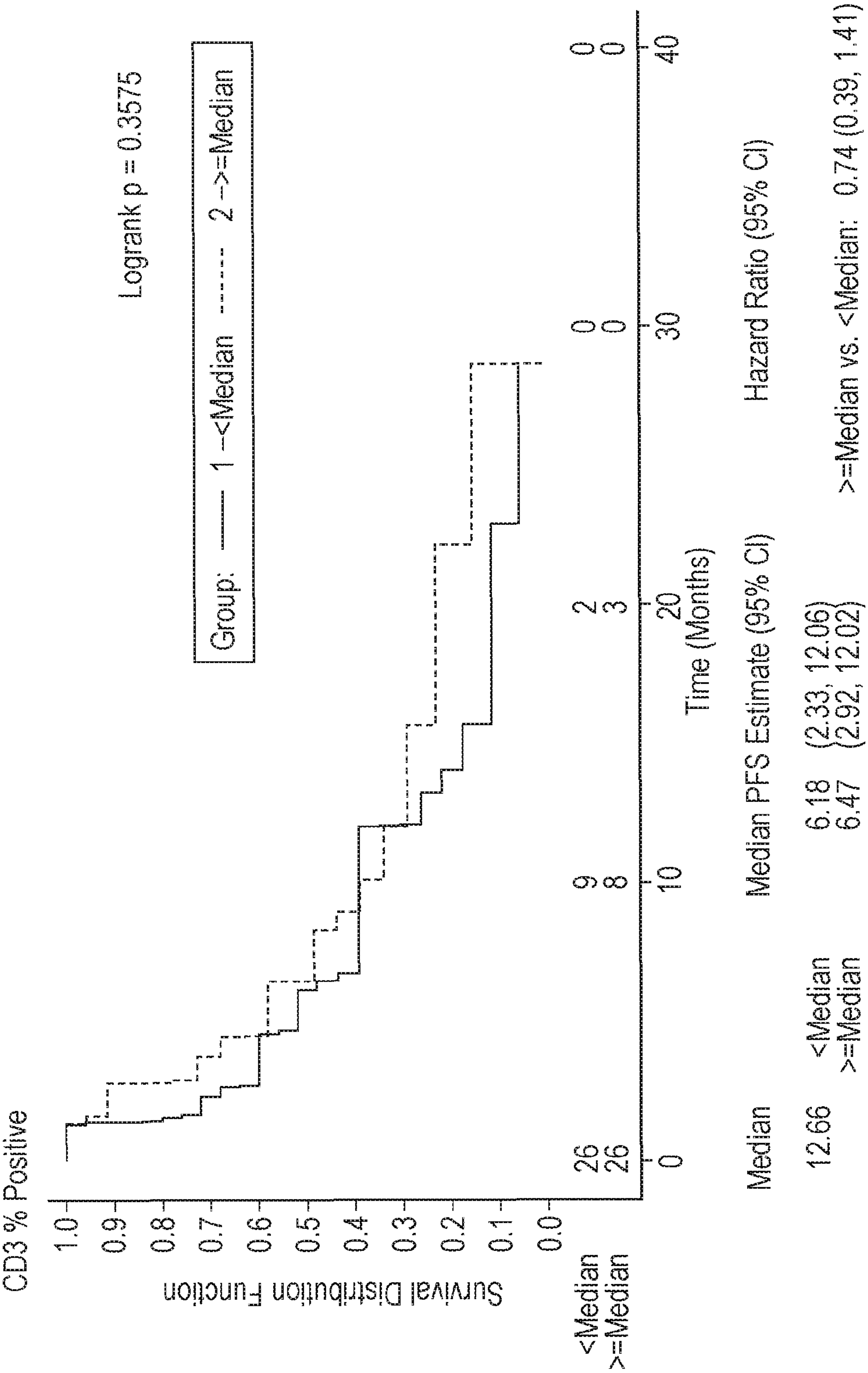
CD3 % Positive
Logrank p = 0.3575
Group: —— 1 -<Median ----- 2 ->=Median
Survival Distribution Function
1.0
0.9
0.8
0.7
0.6
0.5
0.4
0.3
0.2
0.1
0.0
0
10
20
30
40
Time (Months)
<Median 26 9 2 0 0
>=Median 26 8 3 0 0
Median
12.66
<Median
>=Median
Median PFS Estimate (95% CI)
6.18 (2.33, 12.06)
6.47 (2.92, 12.02)
Hazard Ratio (95% CI)
>=Median vs. <Median: 0.74 (0.39, 1.41)

FIG. 6

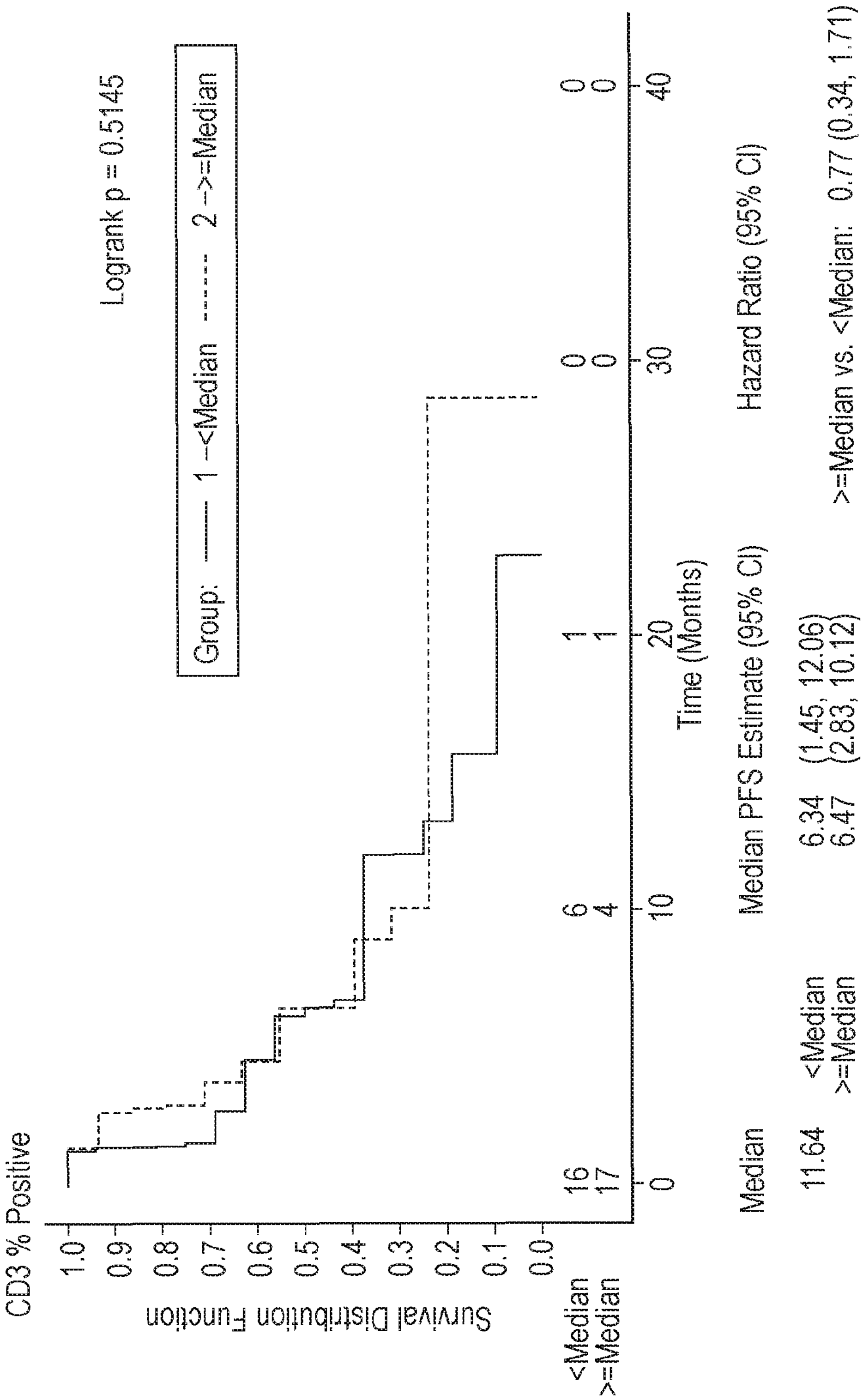
CD3 % Positive
Survival Distribution Function
1.0
0.9
0.8
0.7
0.6
0.5
0.4
0.3
0.2
0.1
0.0
Logrank p = 0.5145
Group: 1 -<Median 2 ->=Median
<Median
>=Median
16 6 1 0 0
17 4 1 0 0
0
10
20
30
40
Time (Months)
Median
11.64
<Median
>=Median
Median PFS Estimate (95% CI)
6.34 (1.45, 12.06)
6.47 (2.83, 10.12)
Hazard Ratio (95% CI)
>=Median vs. <Median: 0.77 (0.34, 1.71)

# FIG. 7

| Variable | Median BM value | Median OS (Months) N | < Median BM Value (95% CI) | N | >= Median BM Value (95% CI) | Logrank p-value | Hazard Ratio (95 % CI) |
|---|---|---|---|---|---|---|---|
| CD3 % Positive | 12.66 | 26 | 20.0 (11.53, 32.36) | 26 | 21.8 (9.33, 23.82) | 0.5668 | 1.222 (0.615, 2.426) |
| CD3 Cell density | 399.50 | 26 | 19.2 (10.61, 32.36) | 26 | 22.6 (13.08, 24.41) | 0.8153 | 0.922 (0.465, 1.826) |
| CD68 % Positive | 5.21 | 26 | 21.8 (11.53, .) | 26 | 20.0 (10.61, 32.36) | 0.9901 | 0.996 (0.497, 1.994) |
| CD68 Cell density | 0.08 | 23 | 17.8 ( 7.03, 24.41) | 29 | 22.6 (13.47, . ) | 0.1733 | 0.619 (0.308, 1.243) |

# FIG. 8

| Variable | Median BM value | N | Median OS (Months): < Median BM Value (95% CI) | N | Median OS (Months): >= Median BM Value (95% CI) | Logrank p-value | Hazard Ratio (95 % CI) |
|---|---|---|---|---|---|---|---|
| CD3 % Positive | 11.64 | 16 | 21.4 (6.87, 32.36) | 17 | 20.0 (5.65, 23.82) | 0.6444 | 1.225 (0.517, 2.900) |
| CD3 Cell density | 334.00 | 16 | 19.2 (10.61, 32.36) | 17 | 21.8 (4.73, 24.41) | 0.6802 | 1.198 (0.508, 2.824) |
| CD68 % Positive | 5.20 | 16 | 18.2 (7.00, .) | 17 | 23.8 (5.65, 32.36) | 0.6576 | 0.818 (0.337, 1.989) |
| CD68 Cell density | 0.06 | 15 | 16.7 (4.27, 21.82) | 18 | 24.1 (10.61, . ) | 0.0777 | 0.451 (0.182, 1.116) |

# FIG. 9

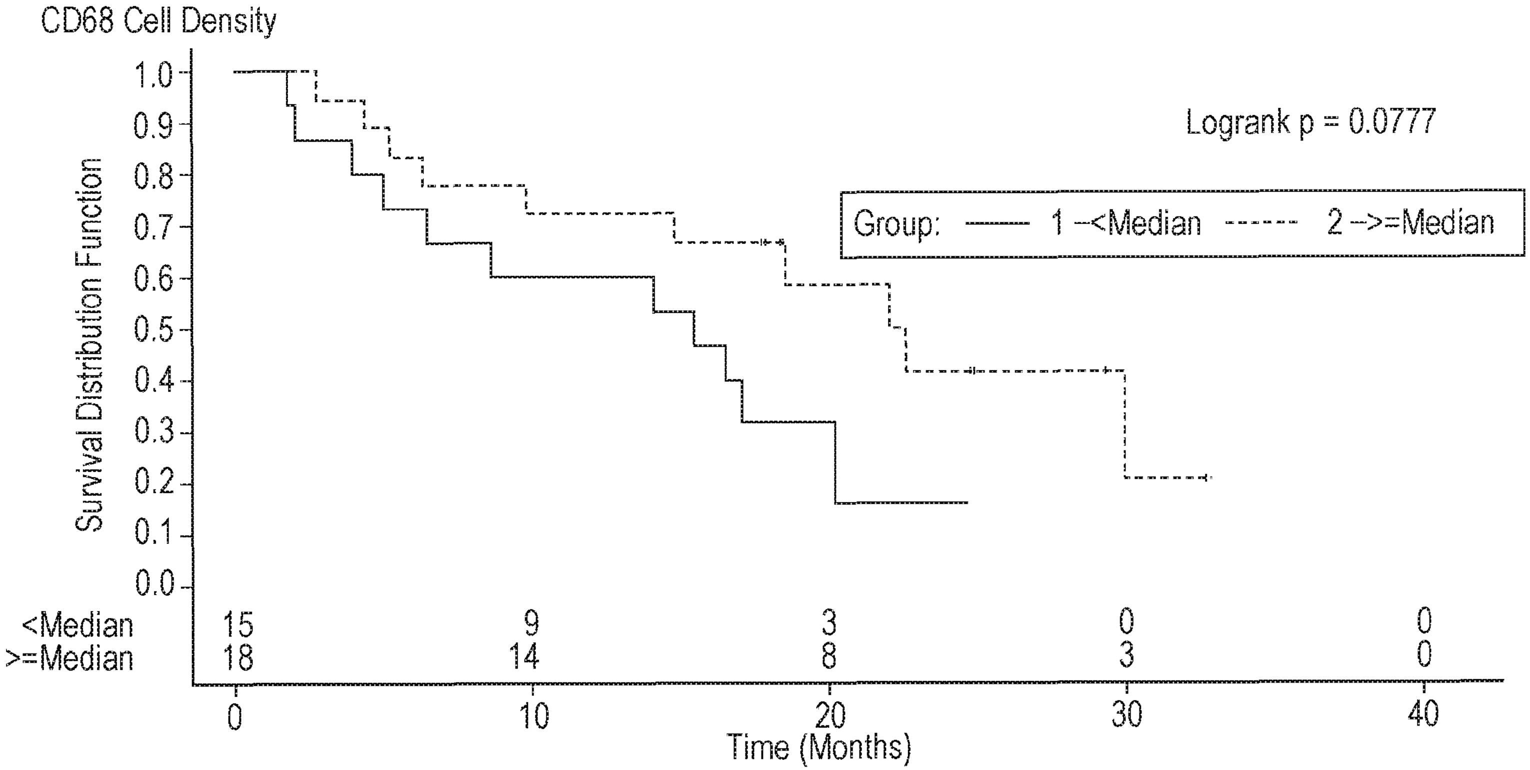


| Median | | Median OS Estimate (95% CI) | | Hazard Ratio (95% CI) | |
|---|---|---|---|---|---|
| 0.06 | <Median | 16.72 | (4.27, 21.82) | | |
| | >=Median | 24.12 | (10.61, .) | >=Median vs <Median: | 0.45 (0.18, 1.12) |

# FIG. 10

| | AG-013736 5.0 mg (BID) | | | |
|---|---|---|---|---|
| Biomarkers | CR+PR N=11 | SD+PD N=33 | *P-Value | Total N=44 |
| CD3 % Positive | | | | |
| n | 11 | 33 | 0.1364 | 44 |
| Mean | 19.54 | 12.93 | | 14.58 |
| Std | 13.168 | 10.265 | | 11.274 |
| %CV | 67.4 | 79.4 | | 77.3 |
| 25th Percentile | 9.20 | 5.00 | | 6.00 |
| Median | 11.20 | 11.60 | | 11.40 |
| 75th Percentile | 30.20 | 19.20 | | 19.90 |
| (Min, Max) | (5.01,44.85) | (0.57,39.15) | | (0.57, 44.85) |
| CD3 Cell density | | | | |
| n | 11 | 33 | 0.174 | 44 |
| Mean | 683.18 | 455.39 | | 512.34 |
| Std | 635.836 | 479.921 | | 524.766 |
| %CV | 93.1 | 105.4 | | 102.4 |
| 25th Percentile | 223.00 | 93.00 | | 162.00 |
| Median | 415.00 | 295.00 | | 356.00 |
| 75th Percentile | 936.00 | 643.00 | | 715.50 |
| (Min, Max) | (159,2241) | (14,2056) | | (14,2241) |
| CD68 % Positive | | | | |
| n | 11 | 33 | 0.0059 | 44 |
| Mean | 10.21 | 4.58 | | 5.98 |
| Std | 6.206 | 4.235 | | 5.328 |
| %CV | 60.8 | 92.6 | | 89.0 |
| 25th Percentile | 4.40 | 1. 30 | | 2.10 |
| Median | 10.02 | 3.73 | | 4.34 |
| 75th Percentile | 15.50 | 6.50 | | 8.30 |
| (Min, Max) | (1.18, 20.66) | (0.2, 20.92) | | (0.2, 20.92) |
| CD68 Cell density | | | | |
| n | 11 | 33 | 0.0071 | 44 |
| Mean | 0.15 | 0.07 | | 0.09 |
| Std | 0.085 | 0.055 | | 0.072 |
| %CV | 58.3 | 82.0 | | 82.4 |
| 25th Percentile | 0.10 | 0.00 | | 0.00 |
| Median | 0.16 | 0.05 | | 0.08 |
| 75th Percentile | 0.20 | 0.10 | | 0.10 |
| (Min, Max) | (0.02, 0.29) | (0, 0.22) | | (0, 0.29) |

*Wilcoxon Rank Sum Test P-value is two-sided t-approximation.

# FIG. 11

| Biomarkers | AG-013736 5.0 mg (BID) CR+PR N=05 | SD+PD N=22 | *P-Value | Total N=27 |
|---|---|---|---|---|
| CD3 % Positive | | | | |
| n | 5 | 22 | 0.1912 | 27 |
| Mean | 17.83 | 11.88 | | 12.98 |
| Std | 10.975 | 9.995 | | 10.235 |
| %CV | 61.6 | 84.1 | | 78.8 |
| 25th Percentile | 9.20 | 4.50 | | 5.00 |
| Median | 11.20 | 8.25 | | 9.16 |
| 75th Percentile | 29.40 | 18.60 | | 19.40 |
| (Min, Max) | (9.15, 30.21) | (0.57, 39.15) | | (0.57, 39.15) |
| CD3 Cell density | | | | |
| n | 5 | 22 | 0.4071 | 27 |
| Mean | 744.20 | 410.23 | | 472.07 |
| Std | 862.342 | 422.772 | | 525.592 |
| %CV | 115.9 | 103.1 | | 111.3 |
| 25th Percentile | 223.00 | 93.00 | | 111.00 |
| Median | 401.00 | 267.50 | | 271.00 |
| 75th Percentile | 697.00 | 643.00 | | 697.00 |
| (Min, Max) | (159,2241) | (14,1744) | | (14,2241) |
| CD68 % Positive | | | | |
| n | 5 | 22 | 0.0407 | 27 |
| Mean | 10.15 | 3.52 | | 4.74 |
| Std | 7.429 | 2.611 | | 4.570 |
| %CV | 73.2 | 74.3 | | 96.4 |
| 25th Percentile | 5.40 | 0.90 | | 1.00 |
| Median | 10.81 | 3.47 | | 3.92 |
| 75th Percentile | 12.70 | 5.20 | | 6.50 |
| (Min, Max) | (1.18, 20.66) | (0.2, 8.71) | | (0.2, 20.66) |
| CD68 Cell density | | | | |
| n | 5 | 22 | 0.0762 | 27 |
| Mean | 0.14 | 0.06 | | 0.07 |
| Std | 0.102 | 0.045 | | 0.066 |
| %CV | 74.0 | 80.8 | | 92.6 |
| 25th Percentile | 0.10 | 0.00 | | 0.00 |
| Median | 0.16 | 0.05 | | 0.05 |
| 75th Percentile | 0.20 | 0.10 | | 0.10 |
| (Min, Max) | (0.02, 0.28) | (0, 0.16) | | (0, 0.28) |

*Wilcoxon Rank Sum Test P-value is two-sided t-approximation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050095634 A, Baker


### Non-patent literature cited in the description


- **SAWYERS.** *Nature,* 2008, vol. 452, 548-552
- **RINI et al.** *Clinical Cancer Research,* 01 June 2011, vol. 17 (11), 3841-9
- **POLIMENO et al.** *BJU Int,* 2013, vol. 112, 686-696
- **SHOJAEI et al.** *Nat. Biotechnol.,* 2007, vol. 25 (8), 911-920
- **LIN et al.** *Eur. J. Cancer Suppl.,* 2010, vol. 8 (7), 191
- **SHOJAEI et al.** *Nat Biotechnol.,* 2007, vol. 25 (8), 911-920
- **TONINI G et al.** *Exper Rev Anticancer Ther,* 2011, vol. 11 (6), 921-930
- **SONPAVDE G ; CHOUEIRI T.** *Br J Cancer,* 2012, vol. 107 (7), 1009-1016
- **AUSUBEL et al.** Current Protocols of Molecular Biology. John Wiley and Sons, 1997
- **CLARK-LANGONE et al.** *BMC Genomics,* 2007, vol. 8, 279
- **DE ANDRES et al.** *Biotechniques,* 1995, vol. 18, 42044
- **ROBERTS et al.** *Laboratory Investigation,* 2007, vol. 87, 979-997
- **ZWEIG et al.** Receiver operating characteristic (ROC) plots: a fundamental evaluation tool in clinical medicine. *Clin. Chem.,* 1993, vol. 39, 561-577
- **PEPE.** The statistical evaluation of medical tests or classification and prediction. Oxford Press, 2003